# EUROPEAN PATENT APPLICATION

(11) **EP 0 857 721 A1**
(43) Date of publication of application: **12.08.1998**
(21) Application number: 96932811.1
(22) Date of filing: 03.10.1996
(51) Int. Cl.: C07D 219/14, C07D 219/06, C07D 401/12, C07D 405/06, C07D 405/12, A61K 31/645, A61K 31/44, A61K 31/47

(54) **ACRIDONE COMPOUNDS**

(30) Priority: 04.10.1995 JP 257944/95; 20.11.1995 JP 301570/95; 06.12.1995 JP 317867/95; 06.12.1995 JP 317868/95; 09.01.1996 JP 1339/96; 09.01.1996 JP 1340/96
(71) Applicant: Eisai Co., Ltd., Tokyo 112-88 (JP)
(72) Inventor: MIYAMOTO, Mitsuaki, Ibaraki 300 (JP); YOSHIUCHI, Tatsuya, Ibaraki 305 (JP); SATO, Keizo, Ibaraki 300-12 (JP); KAINO, Makoto, Ibaraki 305 (JP); TAKASHIMA, Yoshihiro, Ibaraki 305 (JP); MORIYA, Katsuhiro, Ibaraki 305 (JP); SAKUMA, Yoshinori, Ibaraki 300 (JP); YAMADA, Koji, Ibaraki 300-12 (JP); HARADA, kOkichi, Ibaraki 305 (JP); NISHIZAWA, Yukio, Ibaraki 305 (JP); KOBAYASHI, Seiichi, Ibaraki 300 (JP); OKITA, Makoto, Ibaraki 305 (JP); KATAYAMA, Koichi, Ibaraki 305 (JP)
(74) Representative: Hansen, Bernd, Dr. Dipl.-Chem.
(86) International application number: JP9602880
(87) International publication number: WO9712872

(57) **Abstract**

Novel acridone compounds represented by the general formula (I) or pharmaceutically acceptable salts thereof efficacious in the prevention and treatment of diseases in which chemical transmitters such as histamine and leukotriene participate, typified by asthma, allergic rhinitis, atopic dermatitis, urticaria, hay fever, digestive tract allergy and food allergy: 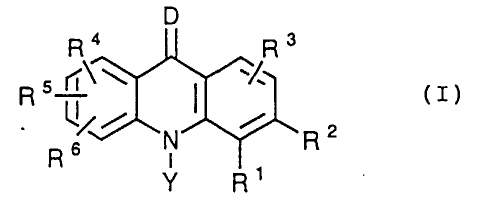 wherein R¹, R², R³, R⁴, R⁵ and R⁶ are the same or different and each represents hydrogen, hydroxy, halogeno, lower alkyl, cycloalkyl, lower alkoxy, etc.; Y represents -(CH₂)ₜ-(B)ₘ-(CH₂)ₙ-Z {wherein m is 0 or 1; t and n represent each an integer of 0 to 6; B represents lower alkylene, optionally substituted arylene, etc.; and Z represents cyano, optionally protected carboxy, acyl, -NR⁷R⁸ [wherein R⁷ and R⁸ are the same or different and each represents hydrogen, lower alkyl, lower alkoxy, hydroxyalkyl, etc.]}; and D represents oxygen or sulfur.

## Description

### Technical Field

This invention relates to novel acridone compounds. More particularly, it relates to novel acridone compounds which are useful as preventives and remedies for diseases against which the effect of preventing an IgE receptor γ-chain from binding to a tyrosine kinase of 72 kDa is efficacious.

### Background Art

Human bronchial asthma and atopic diseases result from various biological reactions complicatedly relating to each other. It is conceived that many of these diseases might be caused in the following manner. Namely, antigen-antibody reactions trigger the liberation of various chemical transmitters from mast cells or basophils. Next, these chemical transmitters contract smooth muscles such as bronchial muscles or pulmonary vessels or elevate the permeability of peripheral vessels, thus causing damages to the living body.

Known examples of the chemical transmitters liberated from mast cells or basophils include histamine, leukotriene, prostaglandin and TNF. It is well known that histamine is the most important chemical transmitter among those described above in relation to the onset of human allergic rhinitis and urticaria. On the other hand, leukotriene, which involves leukotrienes B₄, C₄, D₄, etc., has recently attracted attention in relation to asthmatic attack.

Development of the conventional drugs for preventing the outbreak of allergic diseases or relieving or ameliorating the symptoms thereof aims at inhibiting the production and/or liberation of the above-mentioned chemical transmitters or antagonizing these chemical transmitters.

As a typical example of the drugs developed from this viewpoint, citation may be made of sodium cromoglycate (Intal™) which has been marketed since 1969.

However, the existing antiallergic agents typified by Intal™ show considerable differences between the chemical transmitter-liberation inhibitory concentrations *in vitro* and *in vivo*. Moreover, the sensitivities to these drugs widely vary from patient to patient and the action mechanisms of these drugs are still unclarified in many points.

Mast cells and basophils closely relating to allergic diseases have a highly affinitive receptor Fce RI for the IgE antibody on the cell membrane thereof. When the IgE antibody binds to this receptor and then undergoes crosslinkage with the multivalent antigen corresponding thereto, the intracellular signal transmission mechanism is activated. Thus, histamine is liberated or prostanoids such as leukotrienes and prostaglandins are formed and liberated, which is seemingly associated with the expression of so-called allergic symptoms. On the other hand, it is conceived that the produced cytokines such as TNF and interleukins participate in the chronicity of allergic diseases via interactions with other cells.

### Disclosure of Invention

### Summary of the Invention

An object of the present invention is to provide novel acridone compounds and pharmaceutically acceptable salts thereof, which are efficacious as preventives or remedies for allergic diseases such as asthma, allergic rhinitis, atopic dermatitis, urticaria, hay fever, digestive tract allergy and food allergy.

Another object of the present invention is to provide drugs containing as the active ingredient these acridone compounds or pharmaceutically acceptable salts thereof.

A still another object of the present invention is to provide medicinal compositions comprising these acridone compounds or pharmaceutically acceptable salts thereof and pharmaceutical carriers.

A further object of the present invention is to provide methods for preventing and treating allergic diseases with the use of these acridone compounds or pharmaceutically acceptable salts thereof.

To develop preventives and remedies for allergic diseases, the present inventors have paid their attention to the activation of a tyrosine kinase of non-receptor type of 72 kDa occurring at the early stage in the activation of the intracellular signal transmission mechanism in the process of the liberation of chemical transmitters from mast cells or basophils. It is known that the tyrosine kinase of this type is activated when bonded to the tyrosine activation motif (TAM) containing phosphated tyrosine on the IgE receptor γ-chain. By preventing this binding to thereby inhibit the activation of the tyrosine kinase of 72 kDa, therefore, the activation of the intracellular signal transmission mechanism of mast cells and basophils depending on the IgE antibody can be inhibited and, in its turn, the liberation of the above-mentioned chemical transmitters can be hindered.

From this viewpoint, the present inventors have searched novel compounds capable of preventing the IgE receptor γ-chain from binding to the TAM region. As a result, they have found out that acridone compounds represented by the following general formula (I) and pharmaceutically acceptable salts thereof exhibit the above-mentioned effect, thus completing the present invention.

Accordingly, the first mode of the present invention relates to acridone compounds represented by the general formula (I) or pharmaceutically acceptable salts thereof: 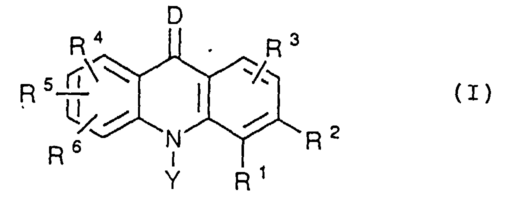 wherein R¹, R², R³, R⁴, R⁵ and R⁶ are the same or different and each represents hydrogen, hydroxy, cyano, nitro, amino, halogeno, lower alkyl, cycloalkyl, cycloalkylalkyl, lower alkoxy, cycloalkyloxy, optionally protected carboxy, optionally substituted heteroaryl, acyl, acylamino, optionally substituted carbamoyl, optionally substituted sulfamoyl, -S(O)ₚ-R⁹ (wherein R⁹ represents hydrogen, lower alkyl or cycloalkyl; and p is an integer of 0 to 2), hydroxyalkyl, alkoxyalkyl, optionally protected and optionally substituted carboxyalkyl, optionally substituted carbamoylalkyl, optionally substituted sulfamoylalkyl, optionally substituted heteroarylalkyl, cyanoalkyl, acylalkyl, acylaminoalkyl, lower alkenyl, hydroxyalkenyl, alkoxyalkenyl, optionally protected carboxyalkenyl, optionally substituted heteroarylalkenyl, cyanoalkenyl, acylalkenyl, acylaminoalkenyl, lower alkynyl, hydroxyalkynyl, alkoxyalkyhyl, optionally protected carboxyalkynyl, optionally substituted heteroarylalkynyl, cyanoalkynyl, acylalkynyl, -W-S(O)_{q}-R¹⁰ (wherein R¹⁰ represents hydrogen, lower alkyl or cycloalkyl; W represents alkylene, alkenylene or alkynylene; and q is an integer of 0 to 2), acylaminoalkynyl, hydroxyalkoxy, alkoxyalkoxy, optionally protected carboxyalkoxy, optionally substituted heteroarylalkoxy, cyanoalkoxy, acylalkoxy, acylaminoalkoxy, or -V-S(O)ᵣ-R¹¹ (wherein R¹¹ represents hydrogen, lower alkyl or cycloalkyl; r is an integer of 0 to 2; and V represents alkyleneoxy),
or two of the substituents R¹, R², R³, R⁴, R⁵ and R⁶ adjacent to each other may form together with the carbon atom to which they are bonded a ring which optionally contains as ring-member atom(s) at least one atom selected from the group consisting of nitrogen, sulfur and oxygen and optionally has substituent(s);
Y represents -(CH₂)ₜ-(B)ₘ-(CH₂)ₙ-Z (wherein m is 0 or 1; t and n represent each an integer of 0 to 6; B represents lower alkylene, lower alkenylene, optionally substituted arylene or optionally substituted lieteroarylene; and Z represents cyano, optionally protected carboxy, optionally substituted carbamoyl, acyl, acylalkyl or -NR⁷R⁸ [wherein R⁷ and R⁸ are the same or different and each represents hydrogen, lower alkyl, lower alkoxy, hydroxyalkyl, alkoxyalkyl, optionally substituted aryl, optionally substituted arylalkyl, optionally substituted heteroaryl, optionally substituted heteroarylalkyl, optionally substituted aryloxy, optionally substituted arylalkoxy, optionally substituted heteroaryloxy, optionally substituted heteroarylalkoxy, optionally protected and optionally substituted carboxyalkyl, acyl, optionally substituted acylamino, optionally substituted acylaminoalkyl, -S(O)ₛ-(X)ᵤ-R¹² (wherein R¹² represents hydrogen, lower alkyl or cycloalkyl; X represents alkylene; s is an integer of 0 to 2; and u is 0 or 1), aminoalkyl, cyanoalkyl, acylalkyl, cycloalkyl, cycloalkylalkyl or amidino optionally substituted by lower alkyl at the N-position, or R⁷ and R⁶ may form together with the nitrogen atom to which they are bonded a ring which optionally contains at least one ring-member factor selected from the group consisting of nitrogen, sulfur, oxygen and >NR⁹ (wherein R⁹ represents hydrogen, lower alkyl, aryl or arylalkyl) and optionally has substituent(s)]}; and
D represents oxygen or sulfur;
   exclusive of the cases where R¹, R², R³, R⁴, R⁵ and R⁶ are the same or different and each represents hydrogen, lower alkyl, lower alkoxy or halogens; Y represents -(CH₂)ₜ-Z (wherein t is an integer of 1 to 6; and Z represents -NR⁷R⁸ [wherein R⁷ and R⁸ are the same or different and each represents hydrogen, lower alkyl, unsubstituted aryl, unsubstituted arylalkyl, unsubstituted heteroaryl or unsubstituted heteroarylalkyl, or R⁷ and R⁸ form together with the nitrogen atom to which they are bonded a five- or six-membered ring further containing as ring-member atom(s) oxygen, sulfur or >NR⁹ (wherein R⁹ is as defined above); and D represent oxygen.

These acridone compounds and pharmaceutically acceptable salts thereof are grouped together except those represented by the general formula (I) and pharmaceutically acceptable salts thereof, wherein R¹, R², R³, R⁴, R⁵ and R⁶ are the same or different and each represents hydrogen, lower alkyl, lower alkoxy or halogens; Y represents -(CH₂)ₜ-Z (wherein t is an integer of 1 to 6; and Z represents -NR⁷R⁸ [wherein R⁷ and R⁸ are the same or different and each represents hydrogen, lower alkyl, optionally substituted aryl, optionally substituted arylalkyl, optionally substituted heteroaryl or optionally substituted heteroarylalkyl, or R⁷ and R⁸ form together with the nitrogen atom to which they are bonded a five-or six-membered ring further containing as ring-member atom(s) oxygen, sulfur or >NR⁹ (wherein R⁹ is as defined above)]}; and D represent oxygen.

Preferable examples of the acridone compounds and pharmaceutically acceptable salts thereof according to the first mode of the present invention are as follows.
1) Compounds represented by the general formula (I) wherein R¹ and R² are the same or different and each represents lower alkyl, or R¹ and R² form together with the carbon atom to which they are bonded a ring which optionally contains as ring-member atom(s) at least one atom selected from the group consisting of nitrogen, sulfur and oxygen and optionally has substituent(s); and R³, R⁴, R⁵, R⁶, Y and D are each as defined above.
2) Compounds represented by the general formula (I) wherein R¹ and R² are both methyl or one of them is methyl while another is lower alkyl; and R³, R⁴, R⁵, R⁶, Y and D are each as defined above.
3) Compounds represented by the general formula (I) wherein R¹, R², R³, R⁴, R⁵, R⁶ and Y are each as defined above; and D is oxygen.
4) Compounds represented by the general formula (I) wherein R¹, R², R³, R⁴, R⁵, R⁶ and Y are each as defined above; and D is sulfur.
5) Compounds represented by the general formula (I) wherein R¹ and R² are both. methyl; R³, R⁴, R⁵, R⁶ and D are each as defined above; and Y is -(CH₂)ₜ-Z {wherein t is an integer of 1 to 3; and Z represents -NR⁷R⁸ [wherein R⁵ and R⁸ are each as defined above]}.
6) Compounds represented by the general formula (I) wherein at least one of R⁴, R⁵ and R⁶ is optionally protected carboxy, optionally protected and optionally substituted carboxyalkyl, optionally protected carboxyalkenyl or optionally protected carboxyalkynyl; and R¹, R², R³, Y and D and zero, one or two of R⁴, R⁵ and R⁶ are each as defined above.
7) Compounds represented by the general formula (I) wherein at least one of R⁴, R⁵ and R⁶ is optionally protected carboxy or optionally protected and optionally substituted carboxyalkyl; and R¹, R², R³, Y and D and zero, one or two of R⁴, R⁵ and R⁶ are each as defined above.
8) Compounds represented by the general formula (I) wherein Y represents -CH₂)ₜ-(B)ₘ-(CH₂)ₙ-Z {wherein B, m, n and t are each as defined above; and Z represents -NR⁷R⁸ [wherein one of R⁷ and R⁸ is hydroxylated arylalkyl and another is as defined above]}; and R¹, R², R³, R⁴, R⁵, R⁶ and D are each as defined above;
9) Compounds represented by the general formula (I) wherein R¹ and R² are both methyl; at least one of R⁴, R⁵ and R⁶ is optionally protected carboxy or optionally protected and optionally substituted carboxyalkyl; and R³, Y and D and zero, one or two of R⁴, R⁵ and R⁶ are each as defined above.
10) Compounds represented by the general formula (I) wherein R¹ and R² are both methyl; R⁴ is optionally protected carboxy or optionally protected and optionally substituted carboxyalkyl; and R³, R⁵, R⁶, Y and D are each as defined above.
11) Compounds represented by the general formula (I) wherein R¹ and R² are both methyl; R⁴ is optionally protected carboxy or optionally protected and optionally substituted carboxyalkyl; R³, R⁵, R⁶ and D are each as defined above; and Y represents -(CH₂)ₜ-Z {wherein t is 2 or 3; and Z represents -NR⁷R⁸ [wherein one of R⁷ represents hydrogen or methyl; and R⁸ represents optionally substituted arylalkyl or optionally substituted heteroarylalkyl]}.
12) Compounds represented by the general formula (I) wherein R¹ and R² are both methyl; R⁴ is optionally protected and optionally substituted carboxyalkyl; Y represents -(CH₂)₃-Z (wherein Z represents -NR⁷R⁸ {wherein one of R⁷ represents hydrogen or methyl; and R⁸ represents optionally substituted arylalkyl or optionally substituted heteroarylalkyl]}; and R³, R⁵, R⁶ and D are each as defined above.
13) Compounds represented by the general formula (I) wherein R¹ and R² are both methyl; R³, R⁵ and R⁶ are each hydrogen; R⁴ is optionally protected and optionally substituted carboxymethyl; Y represents -(CH₂)₃-Z {wherein Z represents -NR⁷R⁸ [wherein one of R⁷ represents hydrogen or methyl; and R⁸ represents optionally substituted arylalkyl or optionally substituted heteroarylalkyl]}; and D is as defined above.

The second mode of the present invention relates to agents for preventing an IgE receptory γ-chain from binding to a tyrosine kinase of 72 kDa, which contain as the active ingredient acridone compounds represented by the above general formula (I) or pharmaceutically acceptable salts thereof.

The third mode of the present invention relates to preventives and remedies for diseases against which the effect of preventing an IgE receptor γ-chain from binding to a tyrosine kinase of 72 kDa is efficacious, which contain as the active ingredient acridone compounds represented by the above general formula (I) or pharmaceutically acceptable salts thereof.

The fourth mode of the present invention relates to preventives and remedies for diseases against which an antiallergic effect is efficacious, which contain as the active ingredient acridone compounds represented by the above general formula (I) or pharmaceutically acceptable salts thereof.

The fifth mode of the present invention relates to preventives and remedies for allergic diseases containing as the active ingredient acridone compounds represented by the above general formula (I) or pharmaceutically acceptable salts thereof.

The sixth mode of the present invention relates to preventives and remedies for allergic rhinitis, atopic dermatitis, urticaria, hay fever, digestive tract allergy, food allergy or asthma, which contain as the active ingredient acridone compounds represented by the above general formula (I) or pharmaceutically acceptable salts thereof.

The seventh mode of the present invention relates to medicinal compositions comprising acridone compounds represented by the above general formula (I) or pharmaceutically acceptable salts thereof in an efficacious amount for preventing or treating diseases against which an antiallergic effect is efficacious and pharmacological carriers.

The eighth mode of the present invention relates to methods for preventing or treating diseases against which an antiallergic effect is efficacious, which comprises administering to a patient acridone compounds represented by the above general formula (I) or pharmaceutically acceptable salts thereof in an efficacious dose for preventing or treating the same.

The term "halogeno" as used in the definition of R¹, R², R³, R⁴, R⁵ and R⁶ in the above formula (I) includes fluorine, chlorine, bromine and iodine atoms.

The term "lower alkyl" as used in the definition of R¹, R², R³, R⁴, R⁵, R⁶, R⁷, R⁸, R⁹, R¹⁰, R¹¹ and R¹² means a linear or branched C₁₋₆ alkyl group such as methyl, ethyl, n-propyl, isopropyl, n-butyl, isobutyl, sec-butyl, tert-butyl, n-pentyl, 1,2-dimethylpropyl, 1,1-dimethylpropyl, 2,2-dimethylpropyl, 2-ethylpropyl, n-hexyl, 1,2-dimethylbutyl, 2,3-dimethylbutyl, 1,3-dimethylbutyl, 1-ethyl-2-methylpropyl or 1-methyl-2-ethylpropyl. Hydrogen atoms in these alkyl groups may be substituted by one to three halogen atoms such as fluorine, chlorine, bromine or iodine atoms. Namely, the lower alkyl groups in the formula (I) also include trifluoromethyl, 1,1,1-trifluoroethyl, etc.

The term "lower alkylene" as used in the definition of B means a linear or branched C₁₋₆ alkylene group such as methylene, ethylene, trimethylene, tetramethylene, pentamethylene, propylene, butylene or ethylmethylene. Hydrogen atoms in these alkylene groups may be substituted by one to three halogen atoms such as fluorine, chlorine, bromine or iodine atoms.

The term "lower alkenyl" as used in the definition of R¹, R², R³, R⁴, R⁵ and R⁶ means a linear or branched C₂₋₆ alkenyl group such as vinyl, 1-propenyl, 2-propenyl, isopropenyl, 2-methyl-1-propenyl, 3-methyl-1-propenyl, 2-methyl-2-propenyl, 3-methyl-2-propenyl, 1-butenyl, 2-butenyl or 3-butenyl. The term "lower alkenyl" as used herein also involves those wherein hydrogen atoms in these alkenyl groups are substituted by one to three halogen atoms.

The term "lower alkenylene" as used in the definition of B means a linear or branched C₂₋₆ alkenylene group such as vinylene, propenylene, butenylene, pentenylene, 1-methylvinylene, 1-methylpropenylene, 2-methylpropenylene, 1-methylpentenylene, 3-methylpentenylene, 1-ethylvinylene, 1-ethylpropenylene, 1-ethylbutenylene or 3-ethylbutenylene. The term "lower alkenylene" as used herein also involves those wherein hydrogen atoms in these alkenylene groups are substituted by one to three halogen atoms.

The term "lower alkynyl" as used in the definition of R¹, R², R³, R⁴, R⁵ and R⁶ means a linear or branched C₂₋₆ alkynyl group such as ethynyl, 1-propynyl, 2-propynyl, 1-butynyl, 3-butynyl, 3-methyl-1-propynyl or 2-methyl-3-propynyl. The term "lower alkynyl" as used herein also involves those wherein hydrogen atoms in these alkynyl groups are substituted by one to three halogen atoms.

The term "lower alkynylene" as used in the definition of B means a linear or branched C₂₋₆ alkynylene group such as ethynylene, 1-propynylene, 1-pentynylene, 1-hexynylene, 2-butynylene, 2-pentynylene, 1-methylethynylene, 3-methyl-1-propynylene or 3-methyl-1-butynylene. The term "lower alkynylene" as used herein also involves those wherein hydrogen atoms in these alkynylene groups are substituted by one to three halogen atoms.

The term "cycloalkyl" as used in the definition of R¹, R², R³, R⁴, R⁵, R⁶, R⁷, R⁸, R⁹, R¹⁰, R¹¹ and R¹² means a C₃₋₈ cycloalkyl group such as cyclopropyl, cyclobutyl, cyclopentyl, cyclohexyl, cycloheptyl or cyclooctyl.

The term "cycloalkylalkyl" as used in the definition of R¹, R², R³, R⁴, R⁵, R⁶, R⁷ and R⁸ means one wherein the above-mentioned cycloalkyl is attached to any carbon atom of the above-mentioned lower alkyl.

The term "lower alkoxy" as used in the definition of R¹, R², R³, R⁴, R⁵, R⁶, R⁷ and R⁸ means a linear or branched C₁₋₆ alkoxy group such as methoxy, ethoxy, n-propoxy, isopropoxy, n-butoxy, sec-butoxy, tert-butoxy, n-pentyloxy, 1,2-dimethylpropyloxy, 1,1-dimethylpropyloxy, 2,2-dimethylpropyloxy, 2-ethylpropyloxy, n-hexyloxy, 1,2-dimethylbutyloxy, 2,3-dimethylbutyloxy, 1,3-dimethylbutyloxy, 1-ethyl-2-methylpropyloxy or 1-methyl-2-ethylpropyloxy. Hydrogen atoms in these alkoxy groups may be substituted by one to three halogen atoms such as fluorine, chlorine, bromine or iodine atoms. Namely, the lower alkoxy groups in the formula (I) also include monofluoromethoxy, trifluoromethoxy, etc.

The term "cycloalkyloxy" as used in the definition of R¹, R², R³, R⁴, R⁵ and R⁶ means one wherein C₃₋₈ cycloalkyl is attached to an oxygen atom, such as cyclopropyloxy, cyclobutyloxy, cyclopentyloxy, cyclohexyloxy, cycloheptyloxy or cyclooctyloxy.

The term "aminoalkyl" as used in the definition of R⁷ and R⁸ means one wherein amino is attached to any carbon atom in the above-mentioned lower alkyl, such as aminoethyl, 1-aminopropyl, 2-aminopropyl, 1-aminobutyl, 2-aminobutyl, 3-aminobutyl, 2-aminomethylpropyl or 1-aminomethyl.

The term "acyl" as used in the definition of R¹, R², R³, R⁴, R⁵, R⁶, R⁷, R⁸ and Z means a group derived from saturated aliphatic monocarboxylic acids, such as acetyl, propionyl, butyryl, valeryl, isovaleryl or pivaloyl, one derived from unsaturated aliphatic monocarboxylic acids, such as acryloyl, propioloyl, methyacryloyl, crotonoyl or isocrotonoyl, one derived from carbocyclic carboxylic acids, such as benzoyl, naphthoyl, toluoyl, hydroatropoyl, atropoyl or cinnamoyl, one derived from heterocyclic carboxylic acids, such as furoyl, thenoyl, nicotinoyl or isonicotinoyl, one derived from hydroxy carboxylic acids or alkoxy carboxylic acids, such as glycoloyl, lactoyl, glyceroyl, tropoyl, benziloyl, salicyloyl, anisoyl, vanilloyl, piperonyloyl or galloyl, and one derived from various amino acids.

The term "acylalkyl" as used in the definition of R¹, R², R³, R⁴, R⁵, R⁶, R⁷, R⁸ and Z means a group wherein the above-mentioned acyl is attached to any carbon atom in the above-mentioned lower alkyl, such as acetylmethyl, propionylmethyl. benzoylethyl, naphthoylpropyl, cinnamoylpropyl, salicyloylbutyl, nicotinoylpentyl or glyceroylhexyl. Needless to say, the acylalkyl is not restricted to the above-mentioned ones.

The term "acylalkenyl" as used in the definition of R¹, R², R³, R⁴, R⁵ and R⁶ means a group wherein acyl is attached to any carbon atom in the above-mentioned lower alkenyl, such as benzoyl-1-ethylenyl or 2-nicotinoyl-2-propylenyl. Needless to say, the acylalkenyl is not restricted to the above-mentioned ones.

The term "acylalkynyl" as used in the definition of R¹, R², R³, R⁴, R⁵ and R⁶ means a group wherein acyl is attached to any carbon atom in the above-mentioned lower alkynyl.

The term "hydroxyalkyl" as used in the definition of R¹, R², R³, R⁴, R⁵, R⁶, R⁷ and R⁸ means a group wherein hydroxy is attached to any carbon atom in the above-mentioned lower alkyl, such as hydroxymethyl, 1-hydroxyethyl, 2-hydroxyethyl, 1-hydroxypropyl, 2-hydroxypropyl or 3-hydroxypropyl. Needless to say, the hydroxyalkyl is not restricted to the above-mentioned ones.

The term "hydroxyalkenyl" as used in the definition of R¹, R², R³, R⁴, R⁵ and R⁶ means a group wherein hydroxy is attached to any carbon atom in the above-mentioned lower alkenyl.

The term "hydroxyalkynyl" as used in the definition of R¹, R², R³, R⁴, R⁵ and R⁶ means a group wherein hydroxy is attached to any carbon atom in the above-mentioned lower alkynyl.

The term "alkoxyalkyl" as used in the definition of R¹, R², R³, R⁴, R⁵, R⁶, R⁷ and R⁸ means a group wherein the above-mentioned lower alkoxy is attached to any carbon atom in the above-mentioned lower alkyl, such as methoxymethyl, ethoxymethyl, ethoxyethyl or 2-ethoxypropyl, though the alkoxyalkyl is not restricted thereto.

The term "alkoxyalkenyl" as used in the definition of R¹, R², R³, R⁴, R⁵ and R⁶ means a group wherein the above-mentioned alkoxy is attached to any carbon atom in the above-mentioned lower alkenyl, such as methoxyethylenyl or ethoxypropylenyl, though the alkoxyalkenyl is not restricted thereto.

The term "alkoxyalkynyl" as used in the definition of R¹, R², R³, R⁴, R⁵ and R⁶ means a group wherein the above-mentioned lower alkoxy is attached to any carbon atom in the above-mentioned lower alkynyl.

The term "cyanoalkyl" as used in the definition of R¹, R², R³, R⁴, R⁵ and R⁶ means a group wherein cyano is attached to any carbon atom in the above-mentioned lower alkyl, such as cyanomethyl, 1-cyanoethyl, 2-cyanoethyl, 1-cyanopropyl or 2-cyanopropyl.

The term "cyanoalkenyl" as used in the definition of R¹, R², R³, R⁴, R⁵ and R⁶ means a group wherein cyano is attached to any carbon atom in the above-mentioned lower alkenyl.

The term "cyanoalkynyl" as used in the definition of R¹, R², R³, R⁴, R⁵, R⁶, R⁷ and R⁸ means a group wherein cyano is attached to any carbon atom in the above-mentioned lower alkynyl.

The term "hydroxyalkoxy" as used in the definition of R¹, R², R³, R⁴, R⁵ and R⁶ means a group wherein hydroxy is attached to any carbon atom in the above-mentioned lower alkoxy, such as hydorxymethoxy, 1-hydroxyethoxy, 2-hydroxyethoxy, 1-hydroxypropoxy, 2-hydroxypropoxy or 3-hydroxypropoxy.

The term "alkoxyalkoxy" as used in the definition of R¹, R², R³, R⁴, R⁵ and R⁶ means a group wherein the above-mentioned lower alkoxy is attached to any carbon atom in the above-mentioned lower alkoxy, such as methoxymethoxy, 1-methoxyethoxy, 2-methoxyethoxy, ethoxymethoxy, 1-ethoxyethoxy, 2-ethoxyethoxy, 1-methoxypropoxy or 2-methoxypropoxy.

The term "cyanoalkoxy" as used in the definition of R¹, R², R³, R⁴, R⁵ and R⁶ means a group wherein cyano is attached to any carbon atom in the above-mentioned lower alkoxy.

The term "acylalkoxy" as used in the definition of R¹, R², R³, R⁴, R⁵ and R⁶ means a group wherein the above-mentioned acyl is attached to any carbon atom in the above-mentioned lower alkoxy.

In the "acylamino" as used in the definition of R¹, R², R³, R⁴, R⁵ and R⁶ and the "optionally substituted acylamino" as used in the definition of R⁷ and R⁸, the term "acyl" has the same meaning as the one of the above-mentioned acyl. The term "acylamino" means one wherein the above-mentioned acyl is attached to the nitrogen atom of the amino group.

In the "acylaminoalkyl" as used in the definition of R¹, R², R³, R⁴, R⁵ and R⁶ and the "optionally substituted acylaminoalkyl" as used in the definition of R⁷ and R⁸, the term "acylaminoalkyl" means one wherein the above-mentioned acylamino is attached to any carbon atom in the above-mentioned lower alkyl.

The term "acylaminoalkenyl" as used in the definition of R¹, R², R³, R⁴, R⁵ and R⁶ means a group wherein the above-mentioned acylamino is attached to any carbon atom in the above-mentioned lower alkenyl.

The term "acylaminoalkynyl" as used in the definition of R¹, R², R³, R⁴, R⁵ and R⁶ means a group wherein the above-mentioned acylamino is attached to any carbon atom in the above-mentioned lower alkynyl.

The term "acylaminoalkoxy" as used in the definition of R¹, R², R³, R⁴, R⁵ and R⁶ means a group wherein the above-mentioned acylamino is attached to any carbon atom in the above-mentioned lower alkoxy.

In the "optionally substituted aryl" as used in the definition of R⁷ and R⁸, the term "aryl" means phenyl, 1-naphthyl, 2-naphthyl, anthracenyl, etc. In the "optionally substituted arylene" as used in the definition of B, the term "arylene" means phenylene, naphthylene, anthracenylene, etc.

The term "optionally substituted arylalkyl" as used in the definition of R⁷ and R⁸ means one wherein the above-mentioned aryl is attached to any carbon atom in the above-mentioned lower alkyl.

The term "optionally substituted heteroaryl" as used in the definition of R¹, R², R³, R⁴, R⁵, R⁶, R⁷ and R⁸ means a monovalent group derived from monocycles or fused rings containing one to four atoms selected from the group consisting of sulfur, oxygen and nitrogen atoms in addition to the carbon atoms, such as thienyl, furyl, benzothienyl, benzofuranyl, isobenzofuranyl, pyrrolyl, isothiazolyl, isoxazolyl, tetrazolyl, pyridyl, pyrazinyl, pyridazinyl, indolyl, isoindolyl, quinolyl, isoquinolyl, phthalazyl, quinoxalyl, acridinyl or imidazopyridyl. The term "optionally substituted heteroarylene" as used in the definition of B means a divalent group derived from monocycles or fused rings containing one to four atoms selected from the group consisting of sulfur, oxygen and nitrogen atoms in addition to the carbon atoms.

The term "optionally substituted heteroarylalkyl" as used in the definition of R¹, R², R³, R⁴, R⁵, R⁶, R⁷ and R⁸ means a group wherein the above-mentioned heteroaryl is attached to any carbon atom in the above-mentioned lower alkyl.

The term "optionally substituted heteroarylalkenyl" as used in the definition of R¹, R², R³, R⁴, R⁵ and R⁶ means a group wherein the above-mentioned heteroaryl is attached to any carbon atom in the above-mentioned lower alkenyl.

The term "optionally substituted heteroarylalkynyl" as used in the definition of R¹, R², R³, R⁴, R⁵, R⁶, R⁷ and R⁸ means a group wherein the above-mentioned heteroaryl is attached to any carbon atom in the above-mentioned lower alkynyl.

In the "optionally substituted aryloxy" as used in the definition of R⁷ and R⁸, the term "aryl" has the same meaning as the one of the above-mentioned aryl.

In the "optionally substituted heteroaryloxy" as used in the definition of R⁷ and R⁸, the term "heteroaryl" has the same meaning as the one of the above-mentioned heteroaryl.

The term "optionally substituted arylalkoxy" as used in the definition of R⁷ and R⁸ means one wherein the above-mentioned aryl is attached to any carbon atom in the above-mentioned lower alkoxy.

The term "optionally substituted heteroarylalkoxy" as used in the definition of R¹, R², R³, R⁴, R⁵, R⁶, R⁷ and R⁸ means a group wherein the above-mentioned heteroaryl is attached to any carbon atom in the above-mentioned lower alkoxy.

The term "optionally substituted carbamoyl" as used in the definition of R¹, R², R³, R⁴, R⁵, R⁶ and Z means carbamoyl optionally having one or two substituents on the nitrogen atom.

The term "optionally substituted carbamoylalkyl" as used in the definition of R¹, R², R³, R⁴, R⁵ and R⁶ means a group wherein optionally substituted carbamoyl is attached to any carbon atom in the above-mentioned lower alkyl.

The term "optionally substituted sulfamoyl" as used in the definition of R¹, R², R³, R⁴, R⁵ and R⁶ means sulfamoyl optionally having one or two substituents on the nitrogen atom.

The term "optionally substituted sulfamoylalkyl" as used in the definition of R¹, R², R³, R⁴, R⁵ and R⁶ means a group wherein optionally substituted sulfamoyl is attached to any carbon atom in the above-mentioned lower alkyl.

In any of the optionally substituted aryl, optionally substituted heteroaryl, optionally substituted arylalkyl, optionally substituted heteroarylalkyl, optionally substituted aryloxy, optionally substituted heteroaryloxy, optionally substituted arylalkyloxy, optionally substituted heteroarylalkyloxy, optionally substituted acylamino, optionally substituted acylaminoalkyl, optionally substitutedcarbamoyl, optionally substituted carbamoylalkyl, optionally substituted sulfamoyl, optionally substituted sulfamoylalkyl and optionally protected and optionally substituted carboxyalkyl, examples of the substituents include hydroxy, lower alkyl groups such as methyl, ethyl, n-propyl and isopropyl, halogenated alkyl groups, lower alkoxy groups such as methoxy, ethoxy, n-propoxy and isopropoxy, halogen atoms such as fluorine, chlorine, bromine and iodine, cyano group, acyl groups such as acetyl, propionyl and benzoyl, amino group, nitro group, optionally protected carboxyl groups, acylamino groups, sulfonylamino group, carbamoyl group, sulfamoyl group, aminosulfonyl group, cyanoalkyl groups, hetroaryl groups, carboxyalkyl groups, carboxyalkoxy groups, heteroarylalkyl groups and heteroarylalkoxy groups.

In the "amidino optionally substituted by lower alkyl at the N-position" as used in the definition of R⁷ and R⁸, the lower alkyl substituent has the same meaning as the one defined above.

In the "optionally protected carboxy" as used in the definition of R¹, R², R³, R⁴, R⁵, R⁶ and Z, examples of the protective groups include lower alkyl groups such as methyl, ethyl and tert-butyl, lower alkyl groups substituted by optionally substituted phenyl, such as p-methoxybensyl, p-nitrobenzyl, 3,4-dimethoxybenzyl, diphenylmethyl, trityl and phenethyl, halogenated lower alkyl groups such as 2,2,2-trichloroethyl and 2-iodoethyl, lower alkanoyloxy-substituted lower alkyl groups such as pivaloyloxymethyl, acetoxymethyl, propionyloxymethyl, butyryloxymethyl, valeryloxyemthyl, 1-acetoxyethyl, 2-acetoxyethyl, 1-pivaloyloxyethyl and 2-pivaloyloxyethyl, higher alkanoyloxy-substituted lower alkyl groups such as palmitoyloxyethyl heptadecanoyloxymethyl and 1-palmitoyloxyethyl, lower alkoxycarbonyloxy-substituted lower alkyl groups such as methoxycarbonyloxymethyl, 1-butoxycarbonyloxyethyl and 1-(isopropoxycarbonyloxy)ethyl, carboxy-substituted lower alkyl groups such as carboxymethyl and 2-carboxyethyl, heteroaryl groups such as 3-phthalidyl, optionally substituted benzoyloxy-substituted lower alkyl groups such as 4-glycyloxybenzoyloxymethyl, (substituted dioxolene)-substituted lower alkyl groups such as (5-methyl-2-oxo-1,3-dioxolen-4-yl)methyl, cycloalkylsubstituted lower alkanoyloxy-substituted lower alkyl groups such as 1-cyclohexylacetyloxyethyl, and cycloalkyloxycarbonyloxy-substituted lower alkyl groups such as 1-cyclohexyloxycarbonyloxyethyl. Moreover, various acid amides fall within the category of the protected carboxy to be used in the present invention. That is to say, the concept of protected carboxy to be used herein involves any group which is decomposed into carboxy by any means *in vivo*.

The term "optionally protected and optionally substituted carboxyalkyl" as used in the definition of R¹, R², R³, R⁴, R⁵, R⁶, R⁷ and R⁸ means a group wherein carboxy is attached to any carbon atom in the above-mentioned lower alkyl. In this case, the carboxy may have the above-mentioned protective group(s) and the alkyl group may have substituent(s) other than carboxy.

The term "optionally protected carboxyalkoxy" as used in the definition of R¹, R², R³, R⁴, R⁵ and R⁶ means a group wherein optionally protected carboxy is attached to any carbon atom in the above-mentioned lower alkoxy. In this case, the protective group has the same meaning as the one defined above.

The term "optionally protected carboxyalkenyl" as used in the definition of R¹, R², R³, R⁴, R⁵ and R⁶ means a group wherein optionally protected carboxy is attached to any carbon atom in the above-mentioned lower alkenyl. In this case, the protective group has the same meaning as the one defined above.

The term "optionally protected carboxyalkynyl" as used in the definition of R¹, R², R³, R⁴, R⁵ and R⁶ means a group wherein optionally protected carboxy is attached to any carbon atom in the above-mentioned lower alkynyl. In this case, the protective group has the same meaning as the one defined above.

The term "pharmaceutically acceptable salts" as used herein means alkali metal salts such as sodium salts, alkaline earth metal salts such as calcium salts, inorganic acid salts such as hydrochlorides, hydrobromides, sulfates and phosphates, organic acid salts such as acetates, maleates, tartrates, methanesulfonates, benzenesulfonates and p-toluenesulfonates, and amino acid salts such as aspartates and glutamates.

The acridone compounds of the present invention and pharmaceutically acceptable salts thereof may be also in the form of hydrates.

The drugs according to the present invention, i.e., the preventives and remedies for various diseases as described above contain as the active ingredient the acridone compounds of the present invention or pharmaceutically acceptable salts thereof.

When the compounds of the present invention are used for preventive or therapeutic purposes, they may be administered orally or parenterally. The compounds of the present invention may be administered in the form of various preparations such as tablets, powders, granules, capsules, syrups, troches, inhalations, suppositories, injections involving those for intravenous drip infusion, ointments, eye ointments, eye drops, nasal drops, ear drops, cataplasmas and lotions.

The administration dose widely varies depending on the type of the disease, the severity of the symptoms, the age, sex and drug-sensitivity of the patient, etc. In general, such a compound is administered to an adult in a daily dose of from about 0.03 to 1,000 mg, preferably from 0.1 to 500 mg and still preferably from 1 to 300 mg, usually one to several times a day. In the case of injection, the invention compound is administered usually in a dose of from about 1 to 3,000 µg/kg body weight, preferably from about 3 to 1,000 µg/kg body weight.

The compounds of the present invention may be processed into preparations by conventional formulation methods with the use of conventional pharmaceutical carriers.

Namely, solid preparations for oral administration are prepared by mixing the invention compounds employed as the principal agent with fillers, binders, disintegrating agents, lubricants, coloring agents, corrigents, antioxidants, etc. Next, the compositions thus obtained are processed into tablets, coated tablets, granules, powders, capsules, etc. each in a conventional manner.

As the above-mentioned fillers, use can be made of lactose, corn starch, sucrose, glucose, sorbitol, crystalline cellulose, silicon dioxide, etc.

As the binders, use can be made of polyvinyl alcohol, polyvinyl ether, ethylcellulose, methylcellulose, acacia, tragacanth, gelatin, shellac, hydroxypropylcellulose, hydroxypropylmethylcellulose, calcium citrate, dextrin, pectin, etc. As the lubricants, use can be made of magnesium stearate, talc, polyethylene glycol, silica, hardened vegetable oils, etc.

As the coloring agents, use can be made of arbitrary ones authorized as pharmaceutical additives. As the corrigents, use can be made of cocoa powder, mentha herb, aromatic powder, mentha oil, borneol, powdered cinnamon bark, etc. As the antioxidant, use can be made of any pharmaceutically authorized ones such as ascorbic acid and α-tocopherol. Needless to say, tablets and granules may be appropriately coated with sugar, gelatin, etc.

On the other hand, injections, eye drops, etc. can be prepared by blending the principal agent with, if needed, pH regulators, buffers, suspending agents, solubilizing agents, stabilizers, tonicity agents, antioxidants, preservatives, etc. and treating the obtained mixtures by conventional methods. If necessary, the obtained preparations may be freeze-dried. These injections can be intravenously, subcutaneously or intramuscularly administered.

Examples of the above-mentioned suspending agents include methylcellulose, polysorbate 80, hydroxyethylcellulose, acacia, tragacanth powder, sodium carboxymethylcellulose and polyoxyethylene sorbitol monolaurate.

Examples of the solubilizing agents include polyoxyethylene-hardened castor oil, polysorbate 80, nicotinamide and polyoxyethylene sorbitol monolaurate.

As the stabilizers, use may be made of sodium sulfite, sodiummetasulfite, ether, etc. As the preservatives, use may be made of methyl p-hydroxybenzoate, sorbic acid, phenol, cresol, chlorocresol, etc.

The medicinal compositions of the present invention including these preparations comprise the acridone compounds according to the present invention or pharmaceutically acceptable salts thereof in an efficacious amount for preventing or treating diseases against which an antiallergic effect is efficacious and at least one of the pharmaceutical carriers as described above.

The methods of the present invention for preventing or treating diseases against which an antiallergic effect is efficacious comprise administering to a patient the acridone compounds according to the present invention or pharmaceutically acceptable salts thereof in an efficacious dose for preventing or treating the same.

The term "efficacious dose for preventing or treating the disease" as used herein varies depending on the particular type of the disease, the severity of the symptoms, the age, sex and drug-sensitivity of the patient, etc.

The compounds of the present invention can be synthesized by combining commonly known methods. Now, main processes for producing these compounds will be described.

### Production process 1

Compounds represented by the formula (I) wherein Z is -NH₂ can be produced through, for example, the following steps 1 to 5.

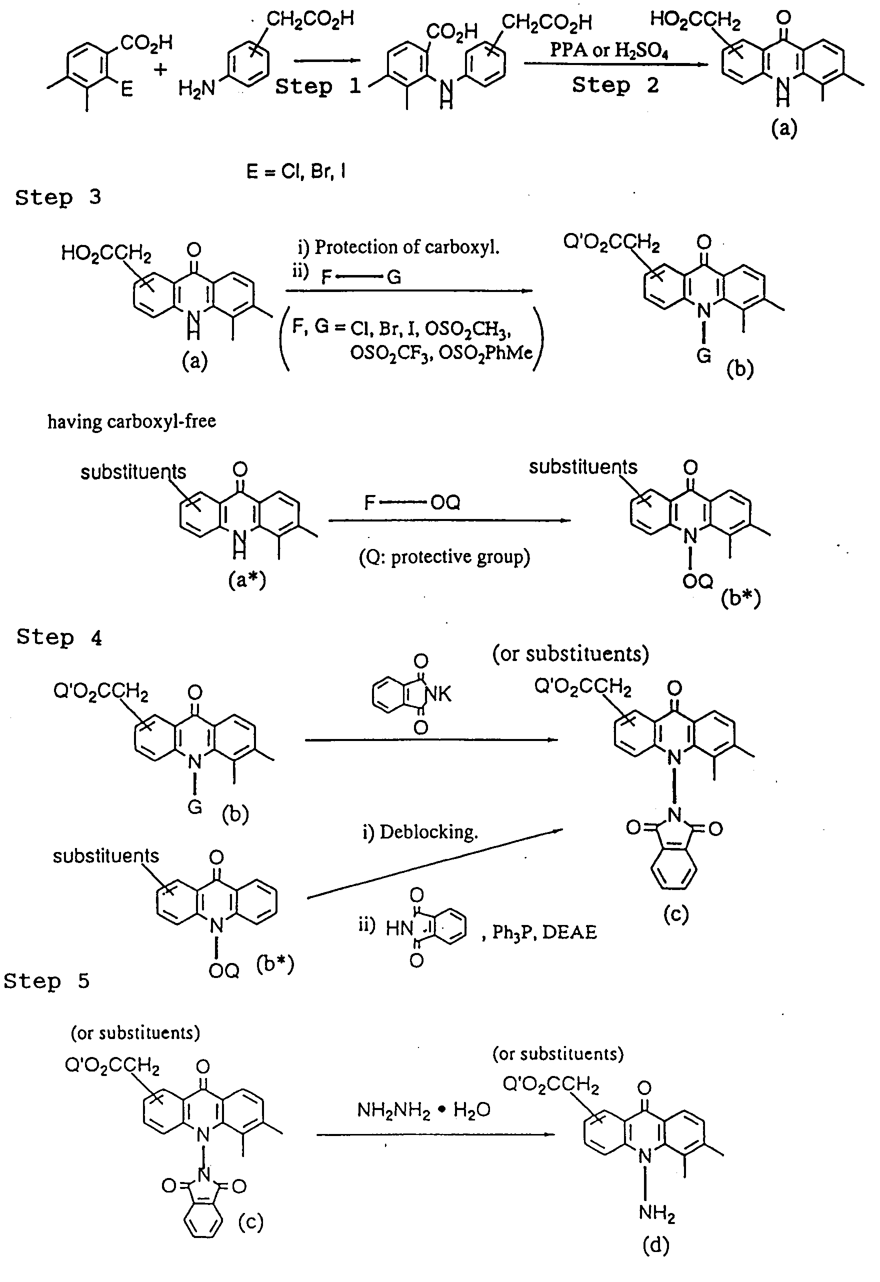

### (Step 1)

The carboxydiphenylamine needed in this step can be synthesized by the Ullmann reaction. More particularly speaking, a mixture of an aniline derivative with a halogenobenzene derivative is dissolved in dimethylformamide. To the solution thus obtained are added potassium carbonate and a catalytic amount of a copper powder and then the resulting mixture is reacted at an elevated temperature of 100°C or higher. The copper powder employed as the catalyst may be replaced by cuprous iodide, while the dimethylformamide employed as the solvent may be replaced by amyl alcohol, nitrobenzene, etc.

### (Step 2)

The cyclization of the carboxydiphenylamine into acridone is effected at room temperature to 80°C with the use of polyphosphoric acid or sulfuric acid.

### (Step 3)

In this step, the nitrogen atom in the compound (a) is metallized. Next, the obtained compound is condensed with an alkyl or aralkyl compound having leaving groups F and G at both ends or an alkyl or aralkyl compound having a leaving group F at one end and an alcoholic hydroxyl group protected with Q at another end. For the metallization, use can be made of butyllithium, lithium diisopropylamide, lithium bistrimethylsilylamide, sodium hydride, potassium hydride, sodium amide, etc. The reaction solvent may be appropriately selected from among inert ones such as diethyl ether, tetrahydrofuran and hexane. The protective group Q may be appropriately selected from among acetyl, tetrahydropyranyl, t-butyldimethylsilyl, t-butyldiphenylsilyl, benzyl, etc. The reaction temperature usually ranges from -20°C to the reflux temperature of the solvent. The leaving groups F and G may be appropriately selected from among halogeno, methanesulfonyl, p-toluenesulfonyl, trifluoromethanesulfonyl, etc.

### (Steps 4 and 5)

Then a nitrogen atom is introduced into the compound (b) synthesized in the step 3 by the Gabriel reaction. Namely, the compound (b) is reacted with potassium phthalimide to thereby give an adduct (c). This reaction is effected usually at 0°C to the reflux temperature of the solvent. The solvent may be appropriately selected from among inert ones such as dimethylformamide, dimethyl sulfoxide, acetone, tetrahydrofuran and methanol.

When a compound (b*) carrying a protected hydroxyl group as a substituent for the leaving group is employed, the compound is deblocked under appropriate conditions. Subsequently, the adduct (c) can be obtained by the Mitsunobu reaction, i.e., by using phthalimide and triphenylphosphine. Then the adduct (c) thus obtained is treated with hydrous hydrazine to thereby give the target compound (d). The reaction temperature is from 0°C to the reflux temperature of the solvent. As the solvent, use may be made of methanol, ethanol, dioxane, etc.

### Production process 2

Compounds represented by the formula (I) wherein Z is -NR⁷R⁸ (provided that at least one of R¹ and R⁸ is not hydrogen) can be produced by, for example, the following process. Step 6

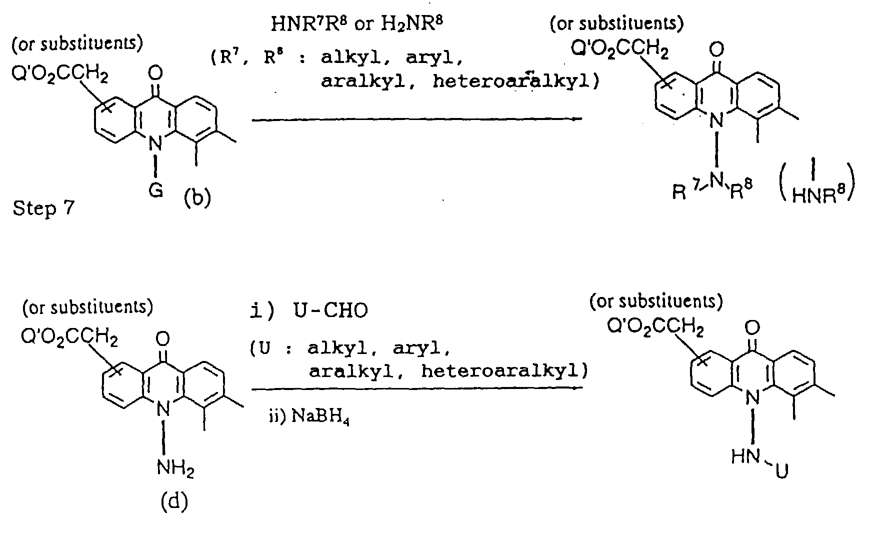

### (Step 6)

The compound (b) synthesized in the step 3 is reacted with a primary or secondary amine optionally in the presence of a solvent at room temperature to the reflux temperature of the solvent. The solvent may be appropriately selected from among inert ones such as methanol, ethanol and tetrahydrofuran.

### (Step 7)

The compound (d) synthesized in the step 5 is subjected to dehydration and condensation with an aldehyde optionally in the presence of a solvent to thereby give a Schiff base. As the solvent, use can be made of benzene, toluene, xylene, ethanol, etc. When benzene, toluene or xylene is employed as the solvent, it is possible to use a water separator. The reaction temperature is from room temperature to the reflux temperature of the solvent.

The Schiff base thus obtained is reduced with sodium boron hydride to thereby give an amine compound. Examples of the solvent usable in this reduction reaction include methanol, ethanol and propanol. The reaction is effected at 0 to 50°C.

To illustrate the usefulness of the acridone compounds according to the present invention and pharmaceutically acceptable salts thereof, a pharmacological experimental example will be given.

### Pharmacological Experimental Example

### (1) Effect of inhibiting liberation of various mediators from rat basophilic leukemia cell line (RBL-2H3)

### i) Experimental method

Under the action of an antigen specific to the IgE antibody, RBL-2H3 cells, i.e., a cell line originating in rat cells, not only liberate histamine and serotonin but also produce and liberate cytokines such as TNF" serving as an inflammatory mediator and prostaglandins. In this experimental system, the effects of the compounds of the present invention of inhibiting the liberation of various mediators were examined by using serotonin as an indication.

The cells were labeled with [³H]-labeled serotonin and, at the same time, sensitized with the IgE antibody. Next, these cells were incubated in the presence of the acridone compounds according to the present invention and then stimulated with the specific antigen. Then the serotonin liberation inhibitory activity of each acridone compound was calculated from the amount of the [³H]-labeled serotonin thus liberated into the medium due to the above-mentioned stimulation and the amount of the [³H]-labeled serotonin liberated into the medium by the same experimental procedure as the one described above but adding no acridone compound of the present invention.

### ii) Results of the experiment

Tables 1 and 2 show the results.

**Table 1**

| Compd. no. | IC₅₀ (µM) in serotonin liberation from RBL-2H3 cells | Compd. no. | IC₅₀ (µM) in serotonin liberation from RBL-2H3 cells |
|---|---|---|---|
| 1 | 3 | 43 | 2 |
| 5 | 3 | 44 | 5 |
| 8 | 8 | 45 | 2 |
| 9 | 10 | 46 | 4 |
| 11 | 0.5 | 47 | 7 |
| 12 | 0.5 | 48 | 1 |
| 13 | 0.2 | 50 | 4 |
| 14 | 10 | 55 | 10 |
| 18 | 10 | 56 | 7 |
| 21 | 8 | 61 | 1 |
| 22 | 3 | 62 | 1 |
| 23 | 8 | 63 | 10 |
| 24 | 8 | 64 | 5 |
| 25 | 6 | 66 | 5 |
| 26 | 10 | 67 | 1 |
| 27 | 10 | 69 | 7 |
| 28 | 1 | 70 | 8 |
| 29 | 8 | 72 | 2 |
| 30 | 2 | 75 | 3 |
| 31 | 1 | 76 | 4 |
| 32 | 1 | 77 | 1 |
| 33 | 2 | 79 | 7 |
| 35 | 3 | 80 | 11 |
| 36 | 2 | 81 | 10 |
| 38 | 2 | 83 | 5 |
| 39 | 1 | 86 | 3 |
| 41 | 3 | 87 | 2 |
| 42 | 2 | 88 | 2 |

**Table 2**

| Compd. no. | IC₅₀ (µM) in serotonin liberation from RBL-2H3 cells | Compd. no. | IC₅₀ (µM) in serotonin liberation from RBL-2H3 cells |
|---|---|---|---|
| 91 | 8 | 119 | 4 |
| 95 | 10 | 120 | 8 |
| 97 | 10 | 125 | 10 |
| 99 | 10 | 126 | 10 |
| 100 | 10 | 127 | 5 |
| 101 | 3 | 128 | 1 |
| 103 | 3 | 129 | 8 |
| 104 | 5 | 130 | 10 |
| 105 | 5 | 131 | 10 |
| 106 | 10 | 133 | 2 |
| 107 | 6 | 135 | 2 |
| 108 | 5 | 136 | 10 |
| 109 | 3 | 140 | 4 |
| 113 | 8 | 141 | 1 |
| 116 | 3 | 142 | 1 |
| 117 | 10 | 143 | 1 |

In the above Tables 1 and 2, the compound numbers correspond to those as will be described hereinafter (the same will apply hereinafter).

### (2) Effect of inhibiting liberation of various mediators from human basophils

### i) Experimental method

To 20 ml of heparinized blood was added 6 ml of 6% dextran (for separating leukocytes, high molecular weight). The obtained mixture was thoroughly stirred and then allowed to stand at 37°C for 30 minutes to thereby sediment erythrocytes. The upper layer was taken out and phosphate-buffered saline was added thereto. The obtained mixture was centrifuged at 185 G for eight minutes to thereby give a crude leukocyte fraction. These cells (leukocyte fraction) were subjected to hypotonic hemolysis and then suspended in D-PBS(+) containing 0.1% of BSA. The cell suspension thus prepared was used in the subsequent experiment as the leukocyte fraction containing basophils.

To 0.4 ml of this cell suspension preliminarily heated at 37°C for 5 minutes was added 0.05 ml of a solution containing each acridone compound of the present invention. The obtained mixture was then allowed to stand at 37°C for 15 minutes so that the cells were pre-treated. Next, 0.05 ml of a tick antigen solution was added thereto to thereby induce an antigen-antibody reaction. Ten minutes thereafter, the antigen-antibody reaction was ceased by ice-cooling. After ceasing the reaction, the mixture was centrifuged at 185 G for ten minutes. The supernatant was taken out and the contents of histamine and a peptide leukotriene therein were determined by using each an enzyme immunoassay kit. Based on the data thus obtained, the activities of the compound of inhibiting the liberation of histamine and peptide leukotriene were determined.

### ii) Results of the experiment

Table 3 shows the results wherein "leukotriene" means the peptide leukotriene.

**Table 3**

| Compd. no. | IC₅₀ (µM) in mediator liberation from human basophils | |
|---|---|---|
| | histamine | leukotriene |
| 1 | 30 | 30-100 |
| 13 | 10-30 | 3-10 |
| 28 | 10 | 3 |
| 39 | 10 | 3 |
| 101 | not measured | 10-30 |
| 135 | not measured | 10-30 |
| 141 | 30-100 | 3-10 |
| 143 | 30 | 10 |

### (3) Effect of preventing IgE receptor γ-chain from binding to tyrosine kinase

### i) Experimental method

In this experiment, use was made of RBL-2H3 cells, i.e., a cell line which is generally employed in the studies on the informational transmission system in IgE receptors of mast cells or basophils and a peptide in the tyrosine activation motif (TAM) region containing phosphorylated tyrosine in a human IgE receptor γ chain synthesized by Peputido Kenkyusho K.K..

In this experiment, use was made of a lysate which had been obtained by solubilizing 1 × 10⁷ to 5 × 10⁷ cells with the use of a solution containing various proteases and NP-40 employed as a solubilizer at a concentration of 1% and a supernatant (as the cytosol fraction of the cells) which had been obtained by disrupting the cells with Down's homogenizer and centrifuging the obtained mixture at 50,000 rpm for 1 hour. The concentrations of the lysate and cytosol of the cells were each adjusted to 1 mg protein/ml with the use of an isotonic buffer.

The phosphorylation experiment of the tyrosine kinase of 72 kDa contained in the lysate or cytosol thus prepared was carried out in the following manner.

First, a test buffer comprising 150 mM of NaCl, 10 mM of KCl, 20 mM of Tris (pH 7.5), 0.6 mM of MnCl₂, 0.5 mM of EGTA, 5 mM of NaF, 1 mM of sodium pyrophosphate and 1 mM of sodium orthovanadate was prepared. To the lysate or cytosol in an amount corresponding to 10 µg of protein were added the above-mentioned test buffer and the compound 101 as will be described in the Example hereinbelow. The obtained mixture was incubated at 30°C for 3 minutes. Then the peptide in the TAM region containing the phosphorylated tyrosine was added so as to give a concentration of 10 µM. Further, ATP was added thereto so as to give a concentration of 50 µM. The resulting mixture was incubated at 30°C for 15 minutes. After the completion of the reaction, the sample was electrophoresed on a 10% agarose gel and the tyrosine kinase of 72 kDa was thus separated.

The extent of the activation of this tyrosine kinase was determined by confirming the extent of the phosphorylation of the tyrosine moiety in the tyrosine kinase per se by western blotting with the use of an anti-tyrosine phosphorylation antibody and then numerically expressing the obtained data with an image analyzer. Based on the extent of the phosphorylation thus obtained, the tyrosine kinase phosphorylation inhibitory ratio of the compound 101 was determined.

### ii) Result of the experiment

Table 4 shows the result of the experiment.

This result clearly indicates that the compound 101 has the effect of preventing the TAM region of the IgE receptor γ-chain from binding to the tyrosine kinase of 72 kDa.

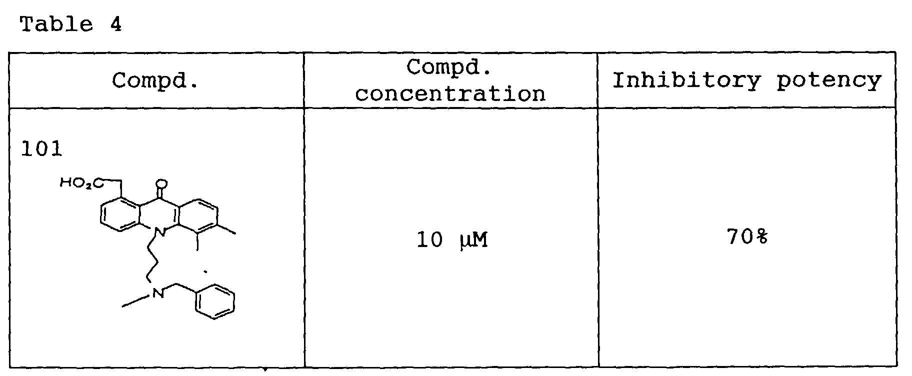

As described above, the compounds of the present invention prevent the IgE receptor γ-chain from binding to the tyrosine kinase of 72 kDa and thus inhibit the activation of the tyrosine kinase to thereby inhibit the liberation of chemical transmitters such as serotonin, histamine and leukotriene. Accordingly, the compounds of the present invention are usable as inhibitors for the binding of the IgE receptor γ-chain to the tyrosine kinase of 72 kDa. Owing to the above-mentioned effect, the compounds of the present invention are usable as preventives and remedies for diseases against which the effect of preventing the IgE receptor γ-chain from binding to the tyrosine kinase of 72 kDa is efficacious and diseases against which an antiallergic effect is efficacious. Moreover, the compounds of the present invention are usable as preventives and remedies for diseases caused by the liberation of chemical transmitters such as serotonin, histamine and leukotriene. More particularly speaking, the compounds of the present invention are useful in preventing and treating allergic diseases typified by asthma, allergic rhinitis, atopic dermatitis, urticaria, hay fever, digestive tract allergy and food allergy.

In addition, the compounds of the present invention are lowly toxic and highly safe, which makes them further useful as drugs.

### Best Mode for Carrying Out the Invention

To further illustrate the present invention, the following Examples will be given, though it is needless to say that the present invention is not restricted thereto.

The compounds synthesized in these Examples are listed in Tables 5 to 34 too. The numbers given at the lower left in the chemical formulae in these Tables mean not the Example numbers but the numbers of the synthesized compounds.

Prior to the presentation of the Examples relating to the synthesis of the compounds of the present invention, Production Examples will be given to illustrate the synthesis of the starting compounds.

### Production Example 1

### 2-(3-Carboxymethylphenyl)amino-3,4-dimethylbenzoic acid:

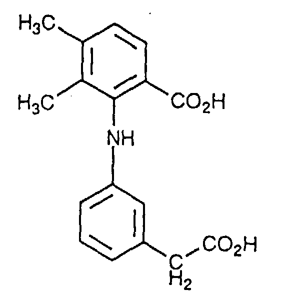

20 g of 3,4-dimethyl-2-iodobenzoic acid [J. Med. Chem., 34, 217 - 222 (1991)], 10.9 g of 3-aminophenylacetic acid, 20 g of potassium carbonate and 500 mg of a copper powder were suspended in 500 ml of N,N-dimethylformamide and the obtained suspension was refluxed under stirring for 2 hours. The mixture thus obtained was cooled to room temperature, neutralized with 1 N hydrochloric acid and then extracted with ethyl acetate. The organic phase was washed with a saturated aqueous solution of sodium chloride and dried over anhydrous magnesium sulfate. The organic phase was distilled under reduced pressure and the solvent was distilled off. The residue (21 g) was not purified but employed as such in the subsequent reaction.

### Production Example 2

### 8-Carboxymethyl-3,4-dimethyl-9(10H)-acridone:

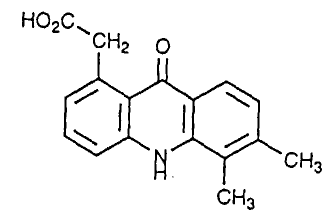

A mixture of 5.4 g of the 2-(3-carboxymethylphenyl)-amino-3,4-dimethylbenzoic acid obtained in Production Example 1 with 70 g of polyphosphoric acid was stirred at 70 to 80°C for 30 minutes. After cooling the mixture to room temperature, ice-water was added thereto. The yellow crystals thus precipitated were separated by filtration and washed with water. The obtained crystals were suspended in dichloromethane and the resulting suspension was refluxed under stirring for 30 minutes. Next, the insoluble matters were filtered off and the filtrate was purified by silica gel column chromatography to thereby give 1.8 g of the title compound.
¹H-NMR(400MHz, DMSO-d₆)δ:
   10.31(s, 1H), 7.92(d, J=8, 1H), 7.82(d, J=8, 1H), 7.55(m, 1H), 7.06(d, J=8, 1H), 6.97(d, J=8, 1H), 4.13(s, 2H), 2.45(s, 3H), 2.40(s, 3H)

From the crystals insoluble in dichloromethane, 2.1 g of 6-carboxymethyl-3,4-dimethyl-9(10H)-acridone was obtained.
¹H-NMR(400MHz, DMSO-d₆)δ:
   10.43(s, 1H), 8.10(d, J=8, 1H), 7.99(d, J=8, 1H), 7.81(s, 1H), 7.11(d, J=8, 1H), 6.99(d, J=8, 1H), 3.71(s, 2H), 2.46(s, 3H), 2.41(s, 3H)

### Production Example 3

### 8-Carboxymethyl-3,4-dimethyl-9(10H)-acridone n-heptyl ester:

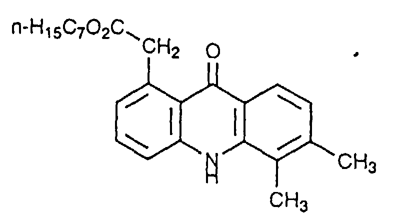

5.1 g of 8-carboxymethyl-3,4-dimethyl-9(10H)-acridone, 4.5 g of caesium carbonate and 3.5 g of brominated n-heptane were dissolved in 100 ml of dimethyl sulfoxide and the obtained solution was stirred at 60°C for 20 minutes. After cooling the solution to room temperature, water was added thereto. Then the mixture was extracted with ethyl acetate. The organic phase was washed with water and dried over anhydrous magnesium sulfate. The organic phase was distilled under reduced pressure and the solvent was distilled off. The residue was purified by silica gel column chromatography to thereby give 4.0 g of the title compound.
¹H-NMR(400MHz, CDCl₃)δ:
   8.27(s, 1H), 7.95(d, J=8, 1H), 7.06(t, J=8, 1H), 6.99(d, J=8, 1H), 6.85(d, J=8, 1H), 6.75(d, J=8, 1H), 4.30(s, 2H), 4.25(t, J=7, 2H), 2.35(s, 3H), 2.25(s, 3H), 1.74(m, 2H), 1.60(m, 2H), 1.40(m, 6H), 0.89(t, J=7, 3H)

### Production Example 4

### 10-(3-Bromopropyl)-8-carboxymethyl-3,4-dimethyl-9-acridone n-heptyl ester:

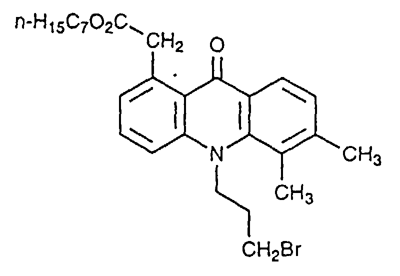

4.0 g of the 8-carboxymethyl-3,4-dimethyl-9(10H)-acridone n-heptyl ester produced in Production Example 3 was dissolved in 200 ml of dry tetrahydrofuran. To the solution thus obtained was added 550 mg of 60% sodium hydride. The resulting mixture was stirred at room temperature for 15 minutes. Then 3.6 g of 3-bromopropyl trifluoromethanesulfonate was added thereto and the obtained mixture was stirred at room temperature for one hour. After adding water, the reaction mixture was extracted with ethyl acetate. The organic phase was washed with water and dried over anhydrous magnesium sulfate. The organic phase was distilled under reduced pressure and the solvent was distilled off. The residue was purified by silica gel column chromatography to thereby give 3.1 g of the title compound.
¹H-NMR(400MHz, CDCl₃)δ:
   8.05(d, J=8, 1H), 7.54(m, 2H), 7.13(d, J=8, 1H), 7.00(m, 1H), 4.42(t, J=7, 2H), 4.22(s, 2H), 4.10(t, J=7, 2H), 2.94(t, J=7, 2H), 2.46(s, 3H), 2.40(s, 3H), 1.80(m, 2H), 1.60(m, 2H), 1.40(m, 8H), 0.86(t, J=7, 3H)

### Production Example 5

### 3-Bromopropyl trifluoromethanesulfonate:

F₃C-S(O)₂-OCH₂CH₂CH₂Br

To a solution of 6.2 g of 3-bromo-1-propanol and 3.8 g of pyridine in dichloromethane (100 ml) was added 7.8 ml of dry trifluoromethanesulfonic acid at 0°C under stirring. The obtained mixture was stirred at 0°C for 10 minutes and then water was added thereto. The organic phase was washed with water and dried over anhydrous magnesium sulfate. The organic phase was distilled under reduced pressure and the solvent was distilled off to thereby give 4.8 g of the title compound.
¹H-NMR(400MHz, CDCl₃)δ:
   4.71(t, J=6, 2H), 3.51(t, J=6, 2H), 2.36(m, 2H)

### Production Example 6

### 8-Carboxymethyl-3,4-dimethyl-10-(3-phthalimidopropyl)-9-acridone n-heptyl ester:

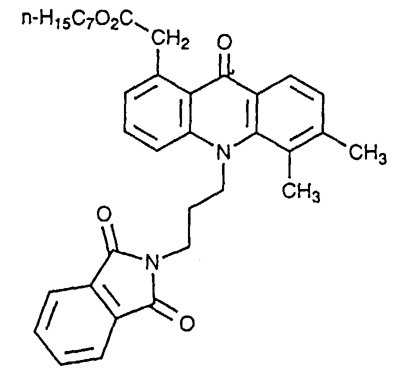

1.2 g of the 10-(3-bromopropyl)-8-carboxymethyl-3,4-dimethyl-9-acridone n-heptyl ester produced in Production Example 4 and 670 mg of phthalimide were dissolved in 30 ml of N,N-dimethylformamide and the obtained solution was stirred at 70°C for 20 minutes. Then the solution was cooled to room temperature. After adding water, the mixture was extracted with ethyl acetate. The organic phase was washed with water and dried over anhydrous magnesium sulfate. The organic phase was distilled under reduced pressure and the solvent was distilled off. Next, the residue was purified by silica gel column chromatography to thereby give 420 mg of the title compound.
¹H-NMR(400MHz, CDCl₃)δ:
   7.98(d, J=8, 1H), 7.76(m, 2H), 7.68(m, 2H), 7.48(m, 2H), 7.05(d, J=8, 1H), 6.96(m, 1H), 4.29(t, J=7, 2H), 4.22(s, 2H), 4.09(t, J=7, 2H), 3.42(t, J=7, 2H), 2.49(s, 3H), 2.43(s, 3H), 1.60(m, 4H), 1.40(m, 8H), 0.84(t, 3=7, 3H)

### Production Example 7

### 3,4-Dimethyl-10-(5-hydroxymethylfuran-2-ylmethyl)-9-acridone:

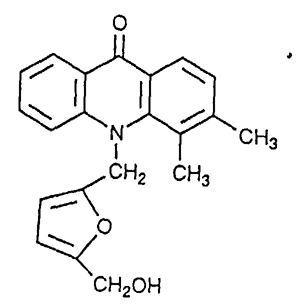

Starting with 3,4-dimethyl-9(10H)-acridone and 2-acetoxymethyl-5-methanesulfonyloxymethylfuran, the procedure of Production Example 4 was repeated to thereby give 10-(5-acetoxymethylfuran-2-ylmethyl)-3,4-dimethyl-9-acridone. 0.32 g of the acridone thus obtained was dissolved in a solvent mixture of 10 ml of tetrahydrofuran with 2 ml of methanol. To the resulting solution was added 3 ml of a 1 N aqueous solution of sodium hydroxide. The obtained mixture was stirred at room temperature for 12 hours. Then the reaction mixture was poured into water. The obtained mixture was extracted with ethyl acetate. The organic phase was washed with water and dried over anhydrous magnesium sulfate. The organic phase was distilled under reduced pressure and the solvent was distilled off. Next, the residue was purified by silica gel column chromatography to thereby give 310 mg of the title compound.
¹H-NMR(400MHz, CDCl₃)δ:
   8.29(d, J=8, 1H), 8.18(d, J=8, 1H), 7.50-7.61(m, 2H), 7.13-7.21(m, 2H), 5.98(d, J=4, 1H), 5.75(d, J=4, 1H), 5.27(s, 2H), 4.33(m, 2H), 2.52(s, 3H), 2.41(s, 3H)

### Production Example 8

### 3,4-Dimethyl-10-(5-phthalimidomethylfuran-2-ylmethyl)-9-acridone:

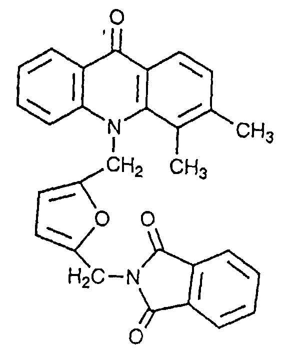

0.15 ml of diethyl azodicarboxylate was added to a solution of 0.31 g of the 3,4-dimethyl-10-(5-hydroxymethylfuran-2-ylmethyl)-9-acridone produced in Production Example 7, 0.25 g of triphenylphosphine and 0.14 g of phthalimide in tetrahydrofuran (5 ml) at 0°C. The obtained mixture was stirred at room temperature for 12 hours. Then the reaction mixture thus obtained was concentrated and the residue was purified by silica gel column chromatography to thereby give 120 mg of the title compound.
¹H-NMR(400MHz, CDCl₃)δ:
   8.18(d, J=8, 1H), 8.09(d, J=8, 1H), 7.85(m, 2H), 7.78(m, 2H), 7.44(m, 2H), 7.02-7.12(m, 2H), 6.08(d, J=4, 1H), 5.65(d, J=4, 1H), 5.23(s, 2H), 4.60(s, 2H), 2.42(s, 3H), 2.40(s, 3H)

### Production Example 9

### 10-(3-Bromopropyl)-8-(1-carboxyethyl)-3,4-dimethyl-9-acridone n-heptyl ester:

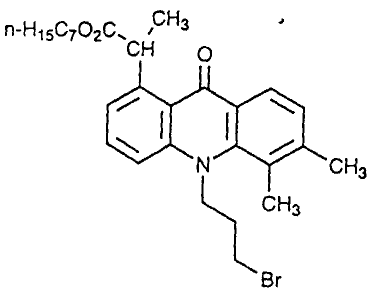

1.0 g of the 10-(3-bromopropyl)-8-carboxymethyl-3,4-dimethyl-9-acridone n-heptyl ester produced in production Example 4 and 1.4 g of iodomethane were dissolved in 20 ml of dimethylformamide. Next, 400 mg of sodium hydride was added to the obtained solution. The resulting mixture was stirred at 60°C for 4 hours. Then the obtained reaction mixture was cooled to room temperature and water was added thereto. The mixture was extracted with ethyl acetate. The organic phase was washed with water and dried over anhydrous magnesium sulfate. The organic phase was distilled under reduced pressure and the solvent was distilled off. Next, the residue was purified by silica gel column chromatography to thereby give 610 mg of the title compound.

### Example 1

### 10-(3-Aminopropyl)-8-carboxymethyl-3,4-dimethyl-9-acridone n-heptyl ester:

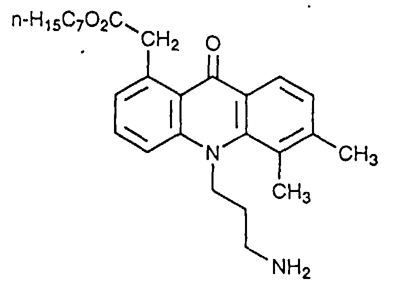

A solution of 420 mg of the 8-carboxymethyl-3,4-dimethyl-10-(3-phthalimidopropyl)-9-acridone n-heptyl ester produced in Production Example 6 and 1.0 g of hydrazine hydrate in methanol (50 ml) was stirred at room temperature for 30 minutes. After adding a saturated aqueous solution of sodium hydrogencarbonate thereto, the obtained mixture was extracted with ethyl acetate. The organic phase was washed with water and dried over anhydrous magnesium sulfate. The organic phase was distilled under reduced pressure and the solvent was distilled off. Next, the residue was purified by silica gel column chromatography to thereby give 210 mg of the title compound.

### Example 2

### 10-(3-Aminopropyl)-8-carboxymethyl-3,4-dimethyl-9-acridone:

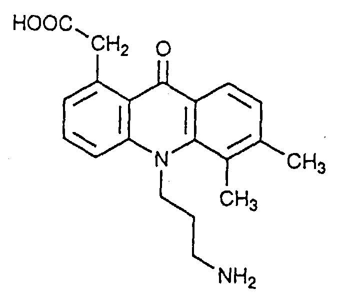

A solution of 210 mg of 10-(3-aminopropyl)-8-carboxymethyl-3,4-dimethyl-9-acridone n-heptyl ester and 200 mg of potassium hydroxide in methanol containing 10% of moisture was stirred at room temperature for 1 hour. After adding water to the resulting solution, the obtained mixture was neutralized with tartaric acid. The obtained solution was purified by reversed phase silica gel column chromatography to thereby give 150 mg of the title compound.

### Example 3

### 10-(3-Benzylaminopropyl)-8-carboxymethyl-3,4-dimethyl-9-acridone:

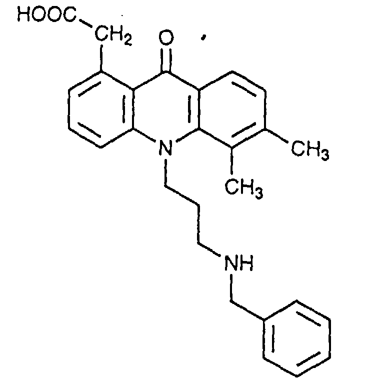

A solution of 120 mg of 10-(3-aminopropyl)-8-carboxymethyl-3,4-dimethyl-9-acridone and 30 mg of benzaldehyde in ethanol (20 ml) was refluxed under stirring for 1 hour. After cooling the obtained mixture to room temperature, 20 mg of sodium boron hydride was added thereto. The obtained mixture was stirred at room temperature for 20 minutes and then water was added thereto. The obtained mixture was neutralized with tartaric acid and the neutralized product was purified by reversed phase silica gel column chromatography to thereby give 120 mg of the title compound.

The compounds listed in Tables 5 and 6 were synthesized by the same procedure as the one of Example 3.

The compounds listed in Table 7 were obtained by the same procedures as those of Examples 1 and 2.

The compounds listed in Table 8 were obtained by the same procedures as those of Examples 1 and 3.

The compounds listed in Tables 9 and 10 were obtained by carrying out the procedures of Examples 1, 3 and 2 in this order. In these cases, the de-esterified products were purified as such in the form of sodium salt.

The compounds listed in Tables 11 to 14 were obtained by the same procedures as those of Examples 1 and 3.

### Example 4

### 3,4-Dimethyl-10-[3-(4-methoxybenzylamino)propyl]-9-acridone:

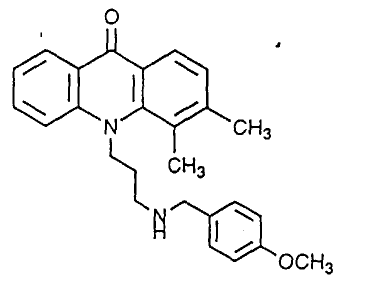

A solution of 500 mg of 10-(3-bromopropyl)-3,4-dimethyl-9-acridone and 360 mg of 4-methoxybenzylamine in ethanol (50 ml) was refluxed under stirring for 1.5 hours. The obtained solution was cooled to room temperature and then a 1 N aqueous solution of sodium hydroxide was added thereto. The obtained mixture was extracted with ethyl acetate. The organic phase was washed with water and dried over anhydrous magnesium sulfate. The organic phase was distilled under reduced pressure and the solvent was distilled off. Next, the residue was purified by silica gel column chromatography to thereby give 280 mg of the title compound.

The compounds listed in Tables 15 to 19 were obtained by the same procedures as the one of Example 4.

The compounds listed in Tables 20 to 22 were obtained by carrying out the procedures of Examples 4 and 2 in this order. In these cases, the de-esterified products were purified as such in the form of sodium salt.

The compounds listed in Table 23 were obtained by the same procedures as those of Examples 1 and 3.

The compounds listed in Table 24 were obtained by carrying out the procedures of Examples 1 and 3 in this order.

### Example 5

### 10-(3-Aminopropyl)-3,4-dimethyl-9-thioacridone:

800 mg of 10-(3-aminopropyl)-3,4-dimethylacridone and 2.9 g of phosphorus pentasulfide were dissolved in 50 ml of pyridine and the obtained solution was stirred at 100°C for 30 minutes. After cooling the reaction mixture to room temperature, water was added thereto. The obtained mixture was extracted with ethyl acetate. The organic phase was washed with water and dried over anhydrous magnesium sulfate. The organic phase was distilled under reduced pressure and the solvent was distilled off. Next, the residue was purified by silica gel column chromatography to thereby give 210 mg of the title compound. Table 25 shows the chemical structural formula and ¹H-NMR data of this compound.

The 10-(3-aminopropyl)-3,4-dimethyl-9-thioacridone obtained in Example 5 was treated in the same manner as the one of Example 3 to thereby synthesize the compound given in Table 26.

### Example 6

The 3,4-dimethyl-10-(5-phthalimidomethylfuran-2-ylmethyl)-9-acridone produced in Production Example 8 was treated in the same manner as the one of Example 1 to thereby synthesize the compound given in Table 27.

### Example 7

By using the compound 132, the compound given in Table 28 was synthesized by the same method as the one of Example 3.

### Example 8

### 8-Aminocarbonylmethyl-3,4-dimethyl-10-(3-N-methylbenzylaminopropyl)-9-acridone:

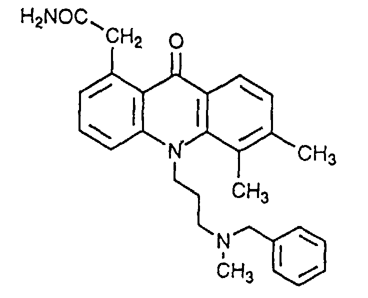

The procedures of Examples 4 and 2 were carried out in this order to thereby give 3,4-dimethyl-8-carboxymethyl10-(N-methylbenzylaminopropyl)-9-acridone. 210 mg of this acridone and 90 mg of 1,1'-carbonyldiimidazole were dissolved in tetrahydrofuran (30 ml). The solution thus obtained was stirred at room temperature for 2 hours. Then 2 ml of a 28% aqueous solution of ammonia was added thereto and the obtained mixture was stirred at room temperature for 1 hour. After adding water thereto, the resulting mixture was extracted with ethyl acetate. The organic phase was washed with water and dried over anhydrous magnesium sulfate. The organic phase was distilled under reduced pressure and the solvent was distilled off. Next, the residue was purified by silica gel column chromatography to thereby give 25 mg of the title compound.

The compound given in Table 29 was synthesized by the same procedure as the one of Example 8.

### Example 9

### 8-Cyanomethyl-3,4-dimethyl-10-(3-N-methylbenzylaminopropyl)-9-acridone:

72 mg of the 8-aminocarbonylmethyl-3,4-dimethyl-10-(N-methylbenzylaminopropyl)-9-acridone obtained in Example 8 and 80 ml of pyridine were dissolved in tetrahydrofuran (20 ml). To the obtained solution was added 70 ml of trifluoromethanesulfonic acid. The mixture thus obtained was stirred at room temperature for 30 minutes and then water was added thereto. The obtained mixture was extracted with ethyl acetate. The organic phase was washed with water and dried over anhydrous magnesium sulfate. The organic phase was distilled under reduced pressure and the solvent was distilled off. Next, the residue was purified by silica gel column chromatography to thereby give 38 mg of the title compound. Table 30 shows the chemical structural formula and ¹H-NMR data of this compound.

### Example 10

The 10-(3-bromopropyl)-8-(1-carboxyethyl)-3,4-dimethyl-9-acridone n-heptyl ester produced in Production Example 9 was treated by the same procedures as those of Examples 4 and 2 to thereby give the compound given in Table 31. In this treatment, the de-esterified product was purified as such in the form of sodium salt.

### Example 11

The 10-(3-bromopropyl)-8-(1-carboxyethyl)-3,4-dimethyl-9-acridone n-heptyl ester produced in Production Example 9 was treated by the same procedures as those of Examples 1, 3 and 2 in this order to thereby give the compound given in Table 32.

### Example 12

### 8-(1-Carboxy-1-methylethyl)-3,4-dimethyl-10-(3-N-methylbenzylaminopropyl)-9-acridone n-heptyl ester:

The procedure of Example 4 was repeated to thereby give 8-(1-carboxyethyl)-3,4-dimethyl-10-(3-N-methylbenzylaminopropyl)-9-acridone n-heptyl ester. 1.3 g of this acridone was dissolved in 50 ml of a solution of 1.5 equivalents of lithium diisopropylamide in dry tetrahydrofuran at -70°C. Then the obtained solution was stirred at -40°C for 30 minutes. To the reaction mixture thus obtained was added a saturated aqueous solution of ammonium chloride. Then the obtained mixture was extracted with ethyl acetate. The organic phase was washed with water and dried over anhydrous magnesium sulfate. The organic phase was distilled under reduced pressure and the solvent was distilled off. Next, the residue was purified by silica gel column chromatography to thereby give 1.2 g of the title compound. Table 33 shows the chemical structural formula and ¹H-NMR data of this compound.

### Example 13

The 8-(1-carboxy-1-methylethyl)-3,4-dimethyl-10-(3-N-methylbenzylaminopropyl)-9-acridone n-heptyl ester obtained in Example 12 was treated in the same manner as the one of Example 2 to thereby synthesize the compound given in Table 34. In this treatment, the de-esterified product was purified as such in the form of sodium salt.

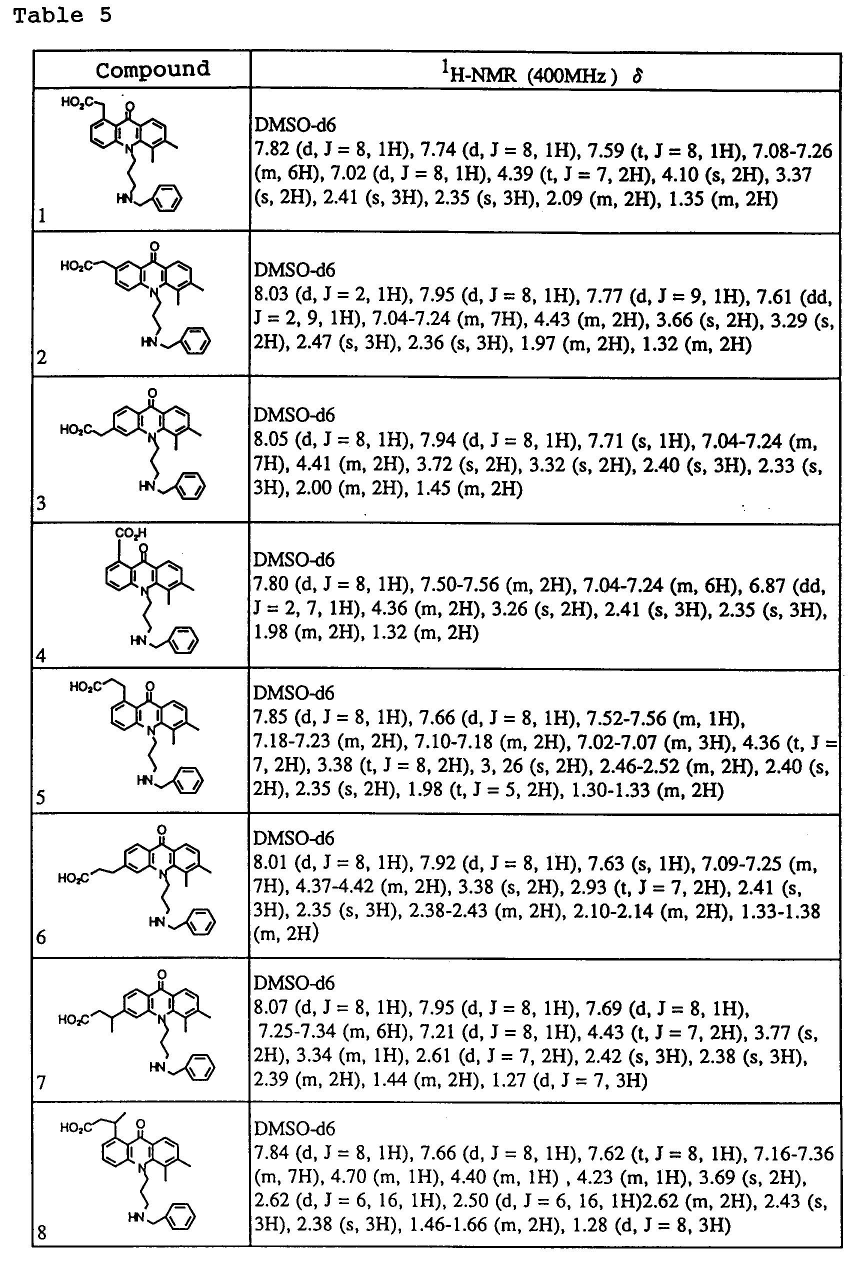

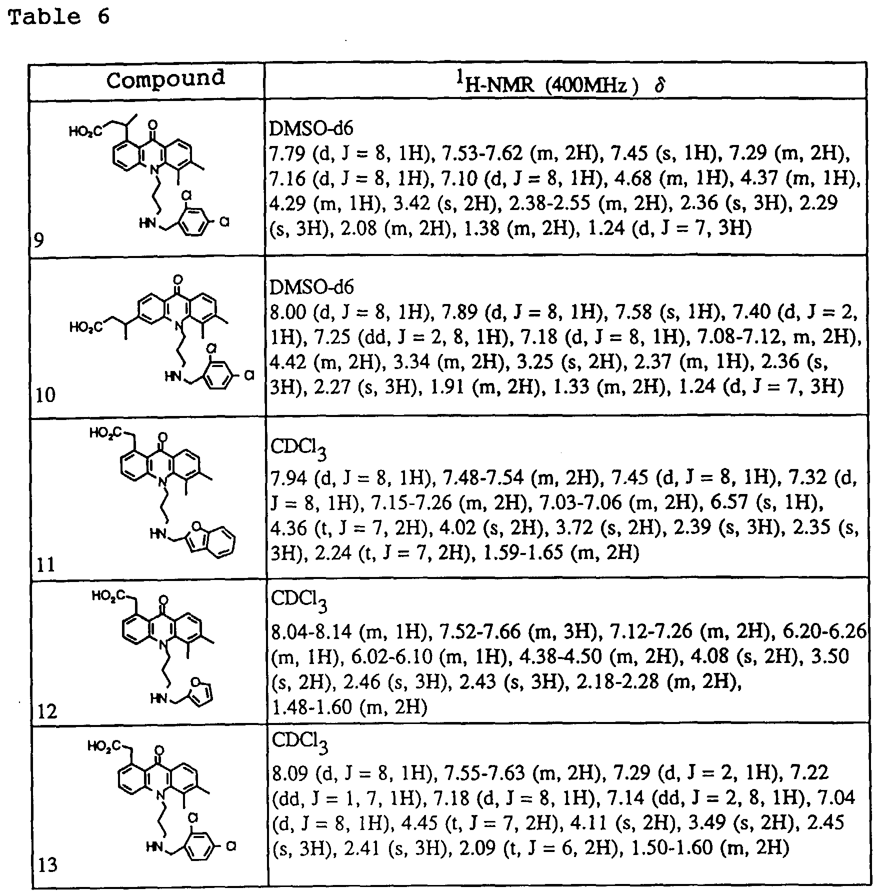

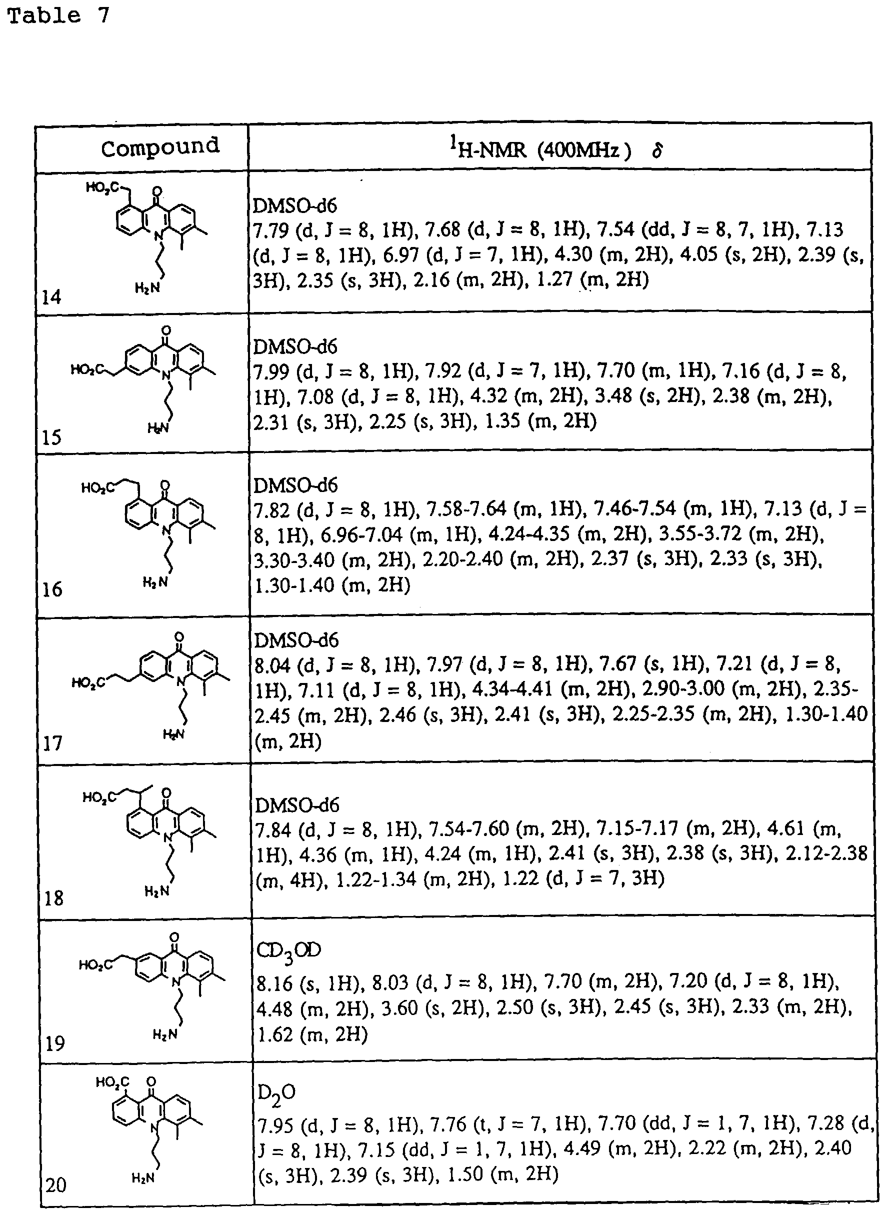

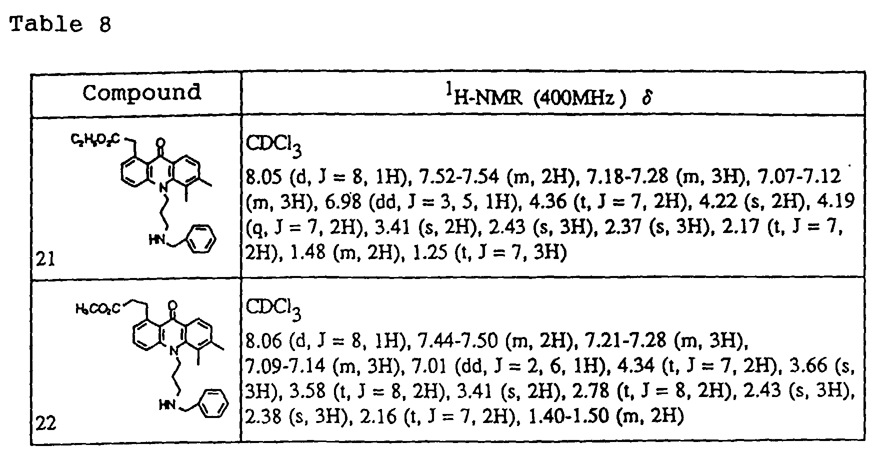

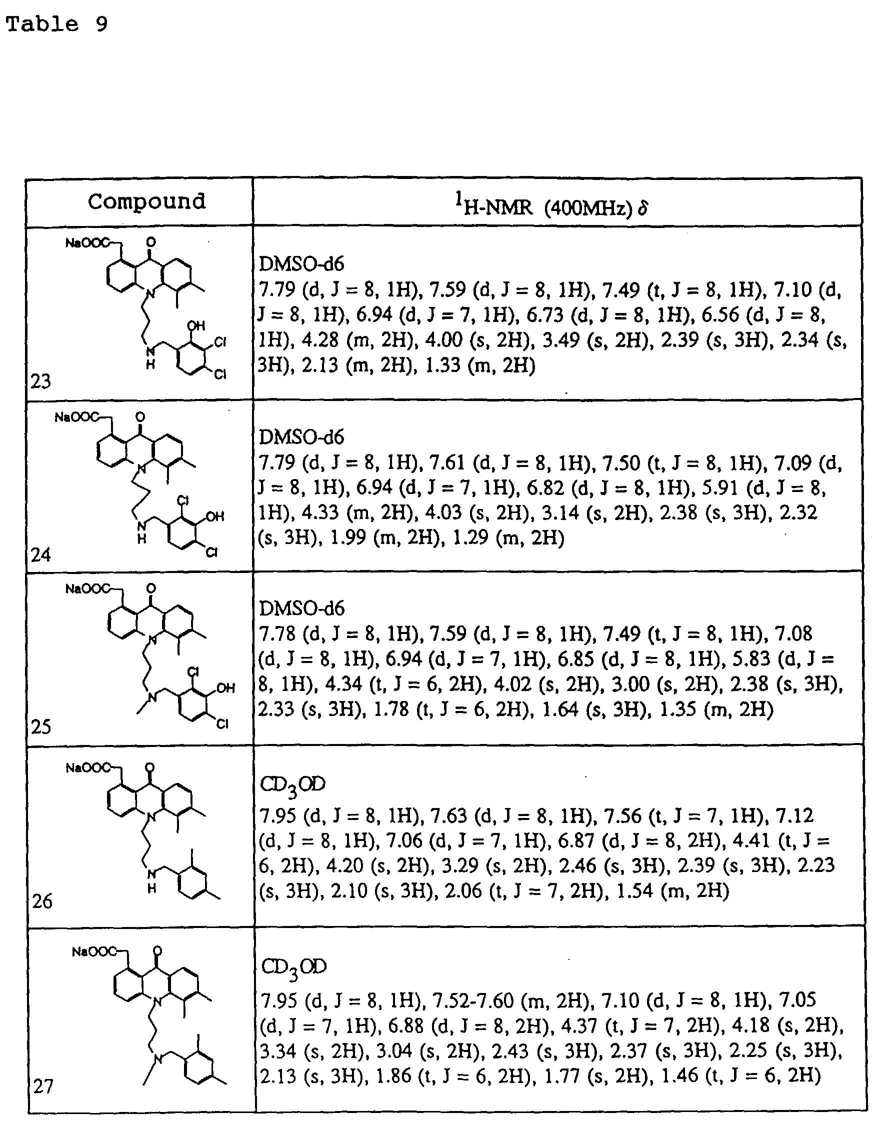

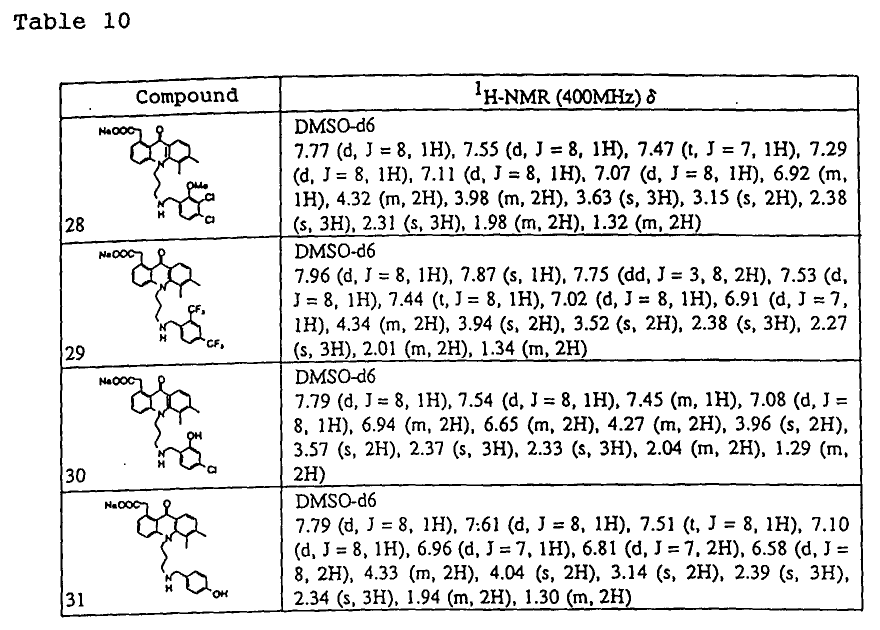

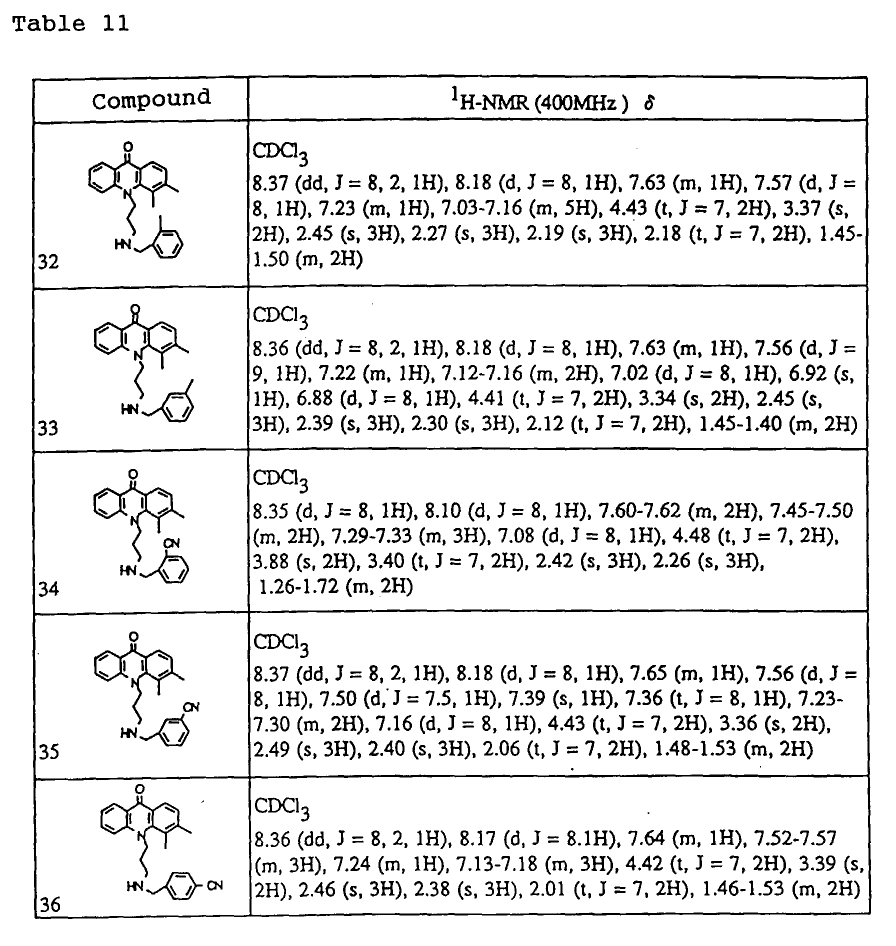

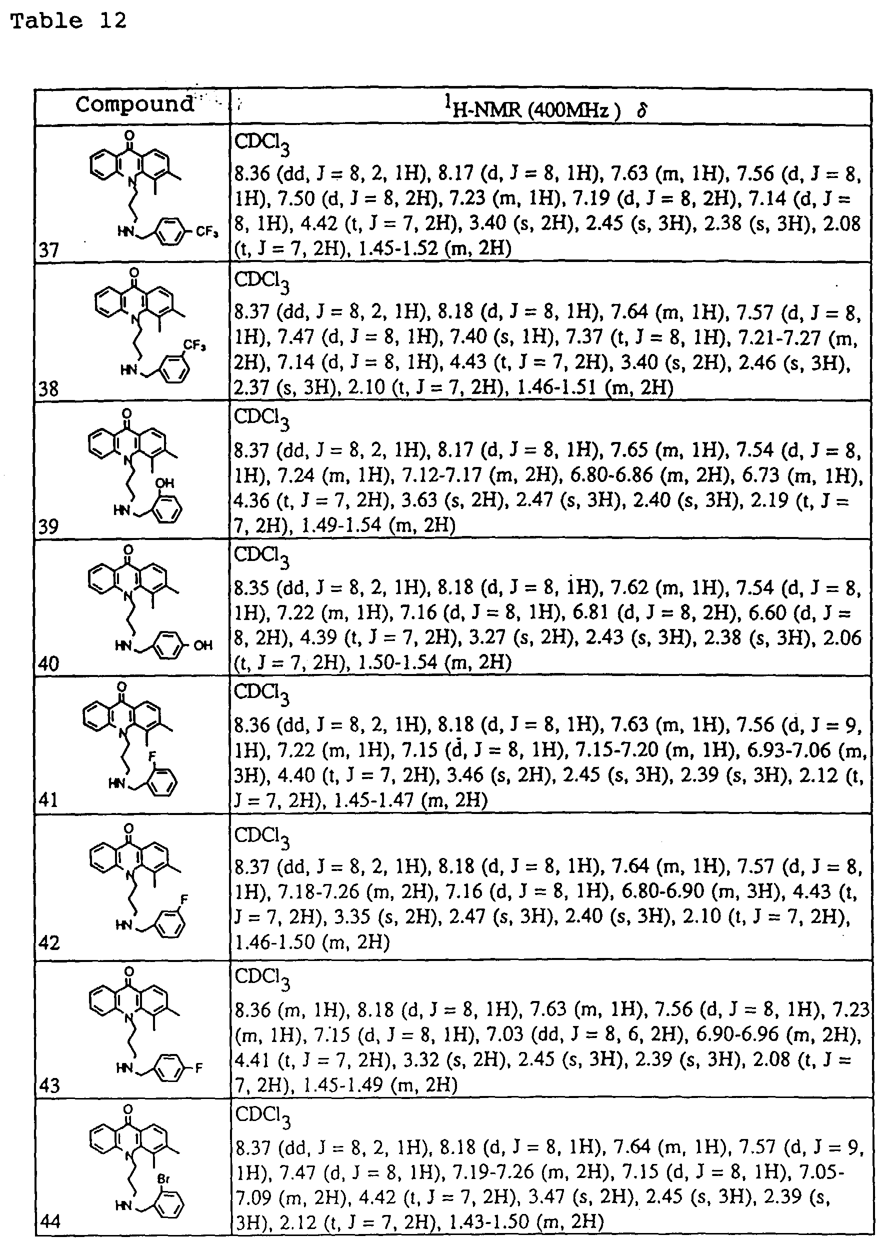

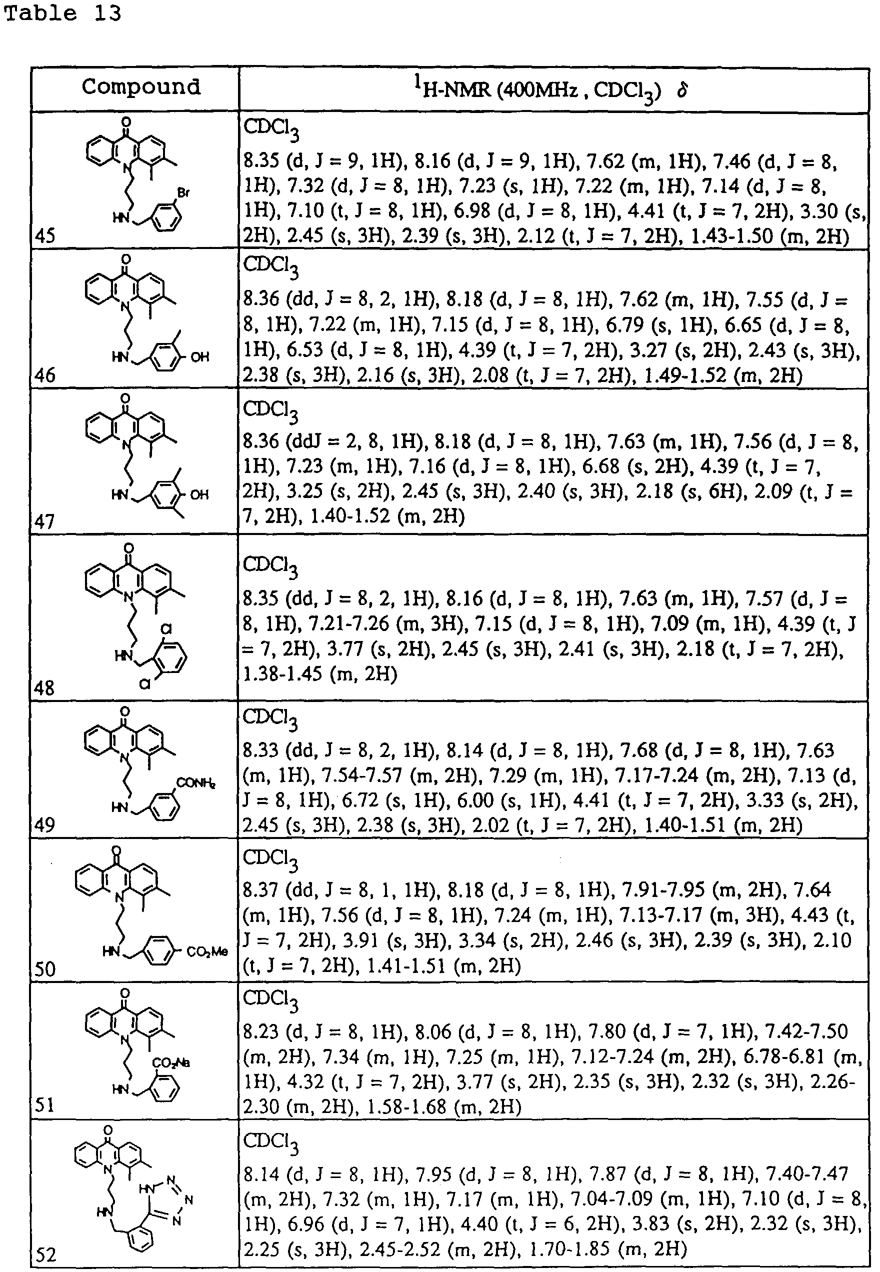

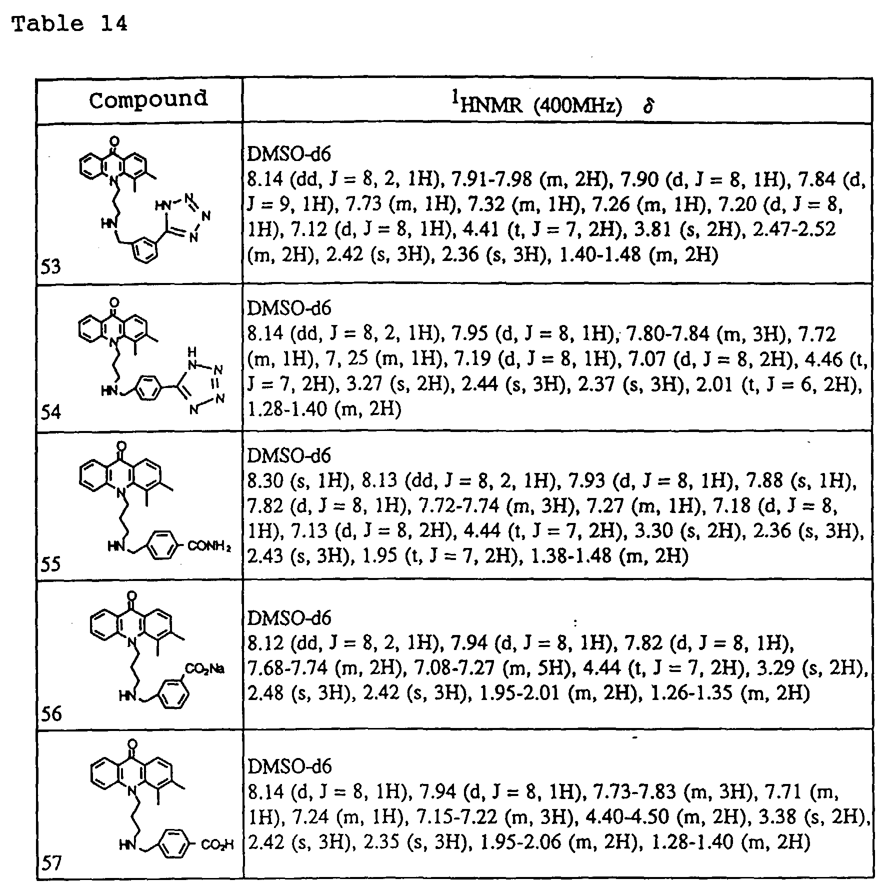

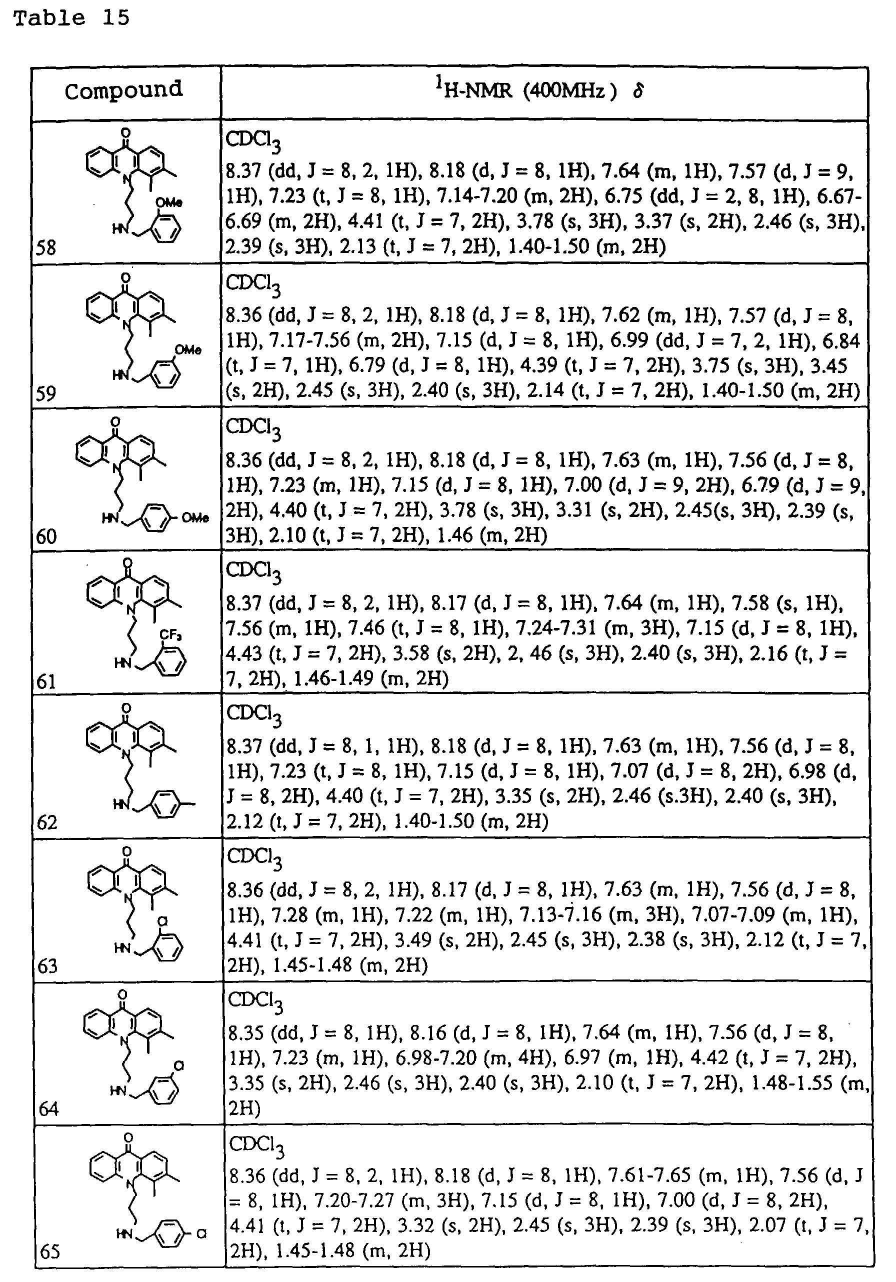

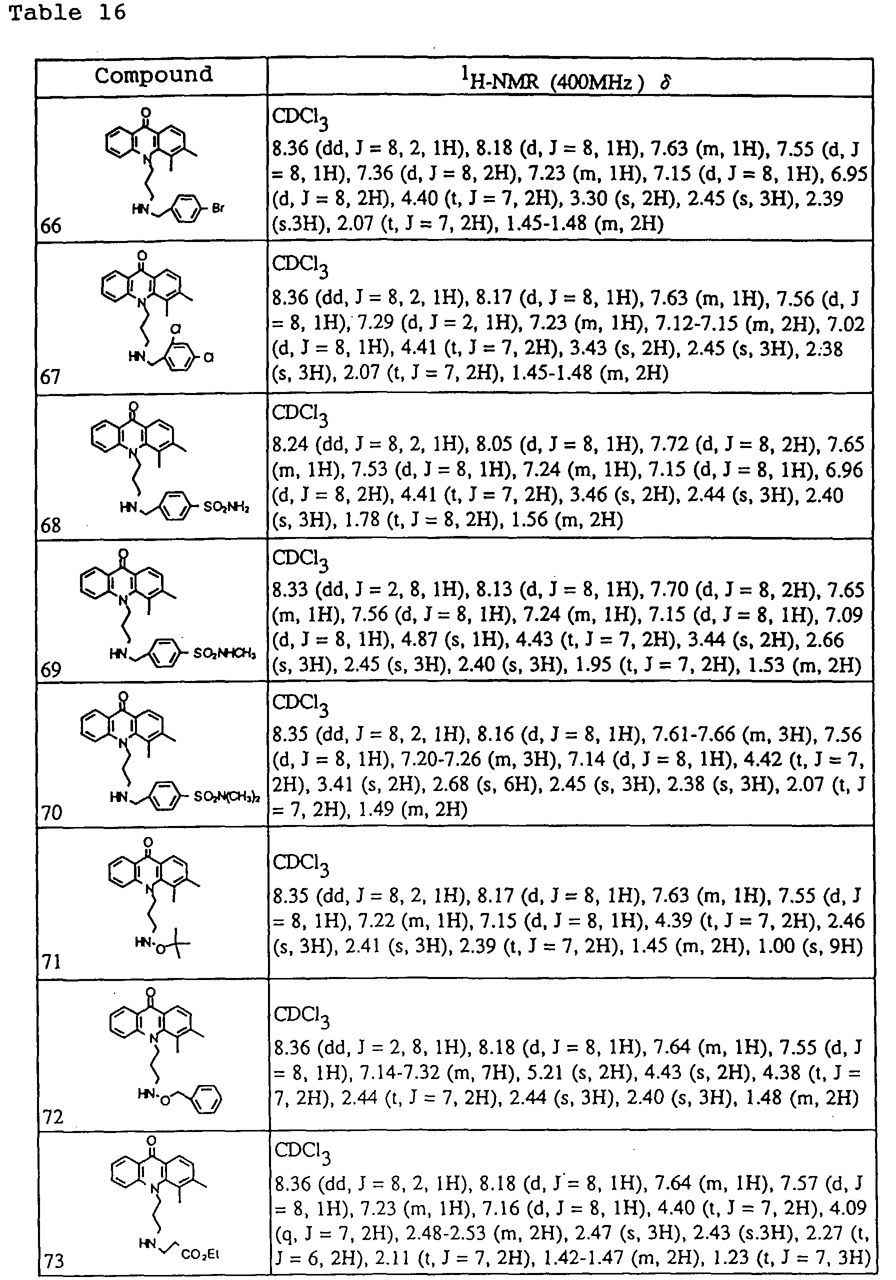

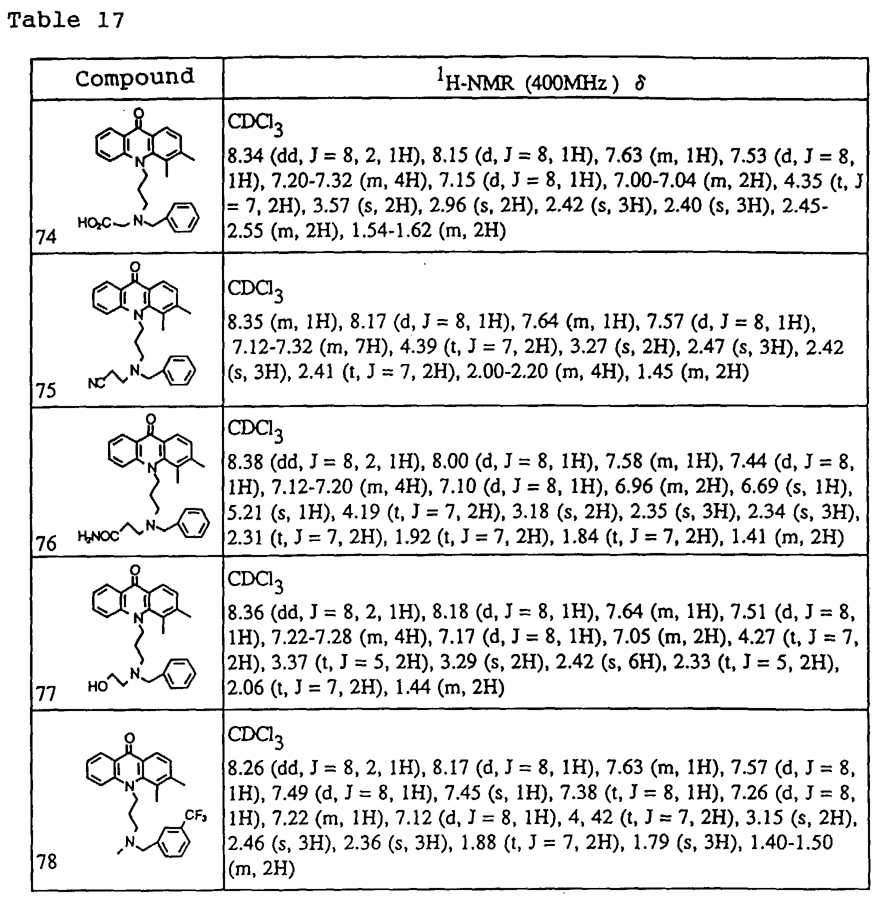

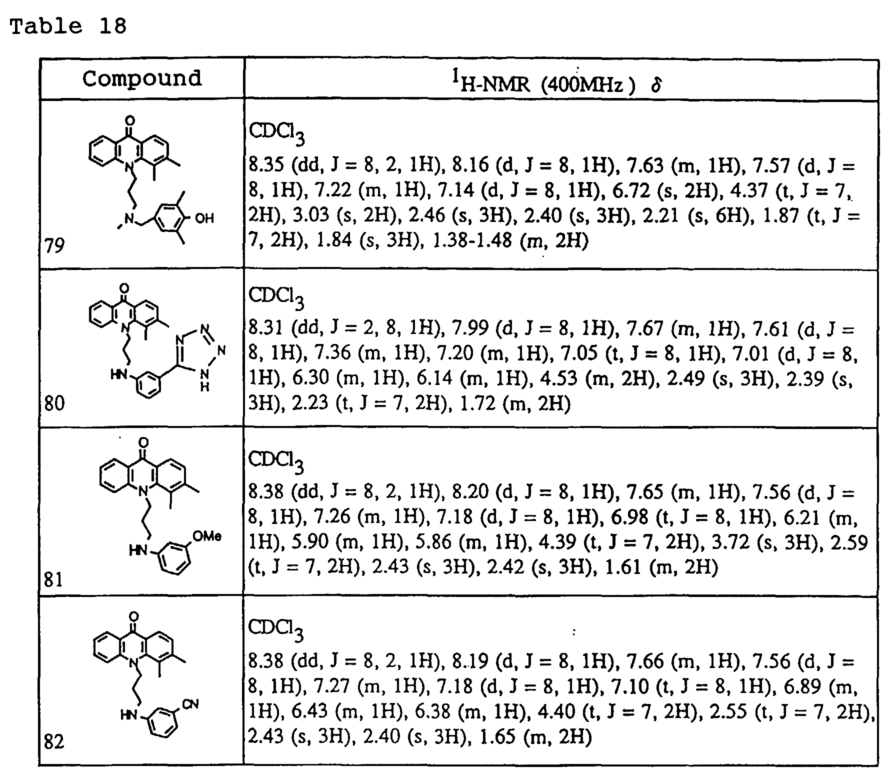

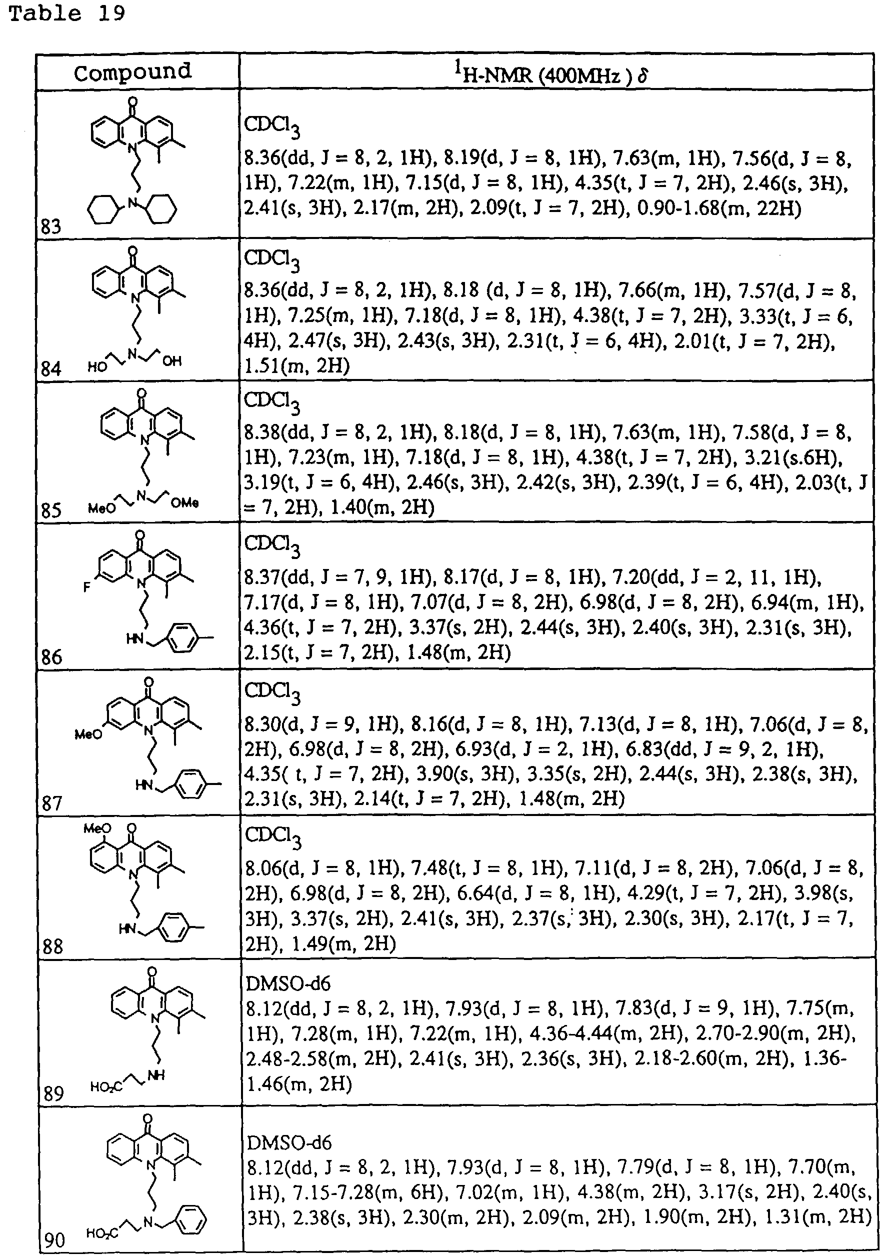

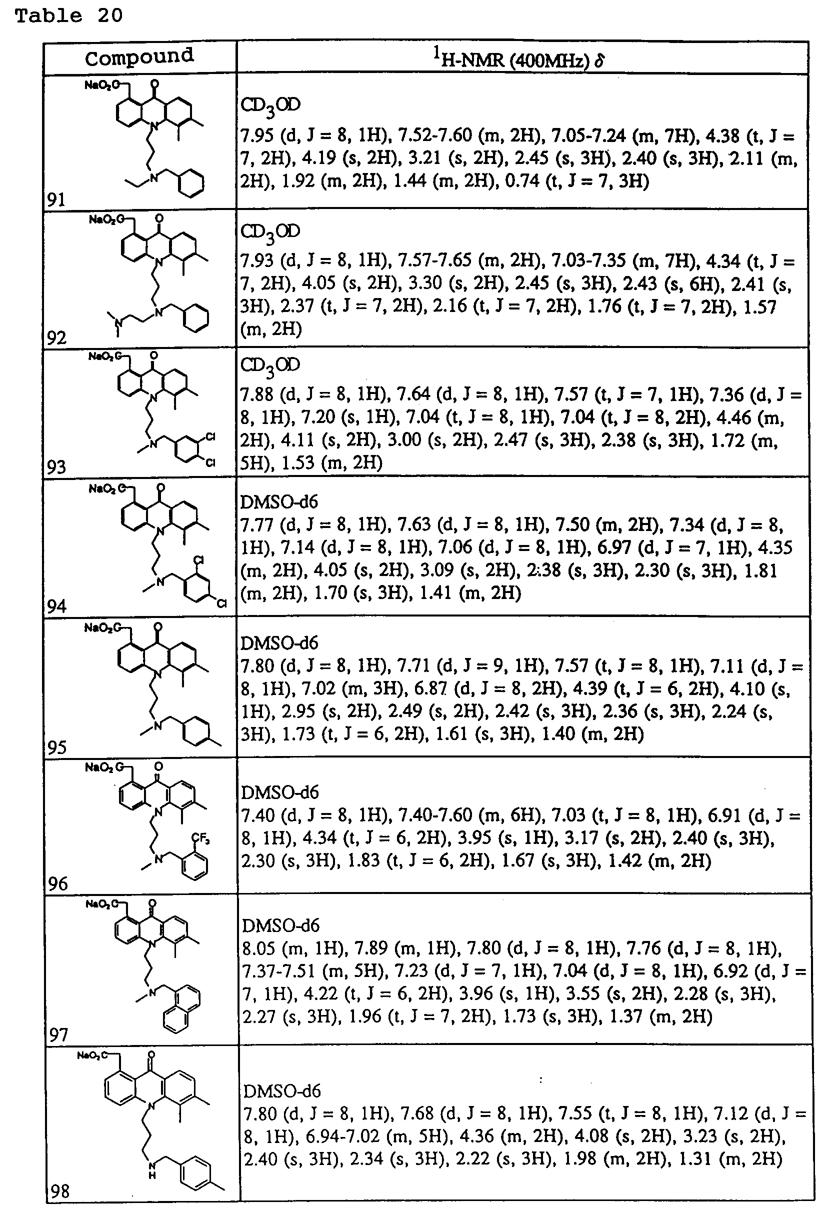

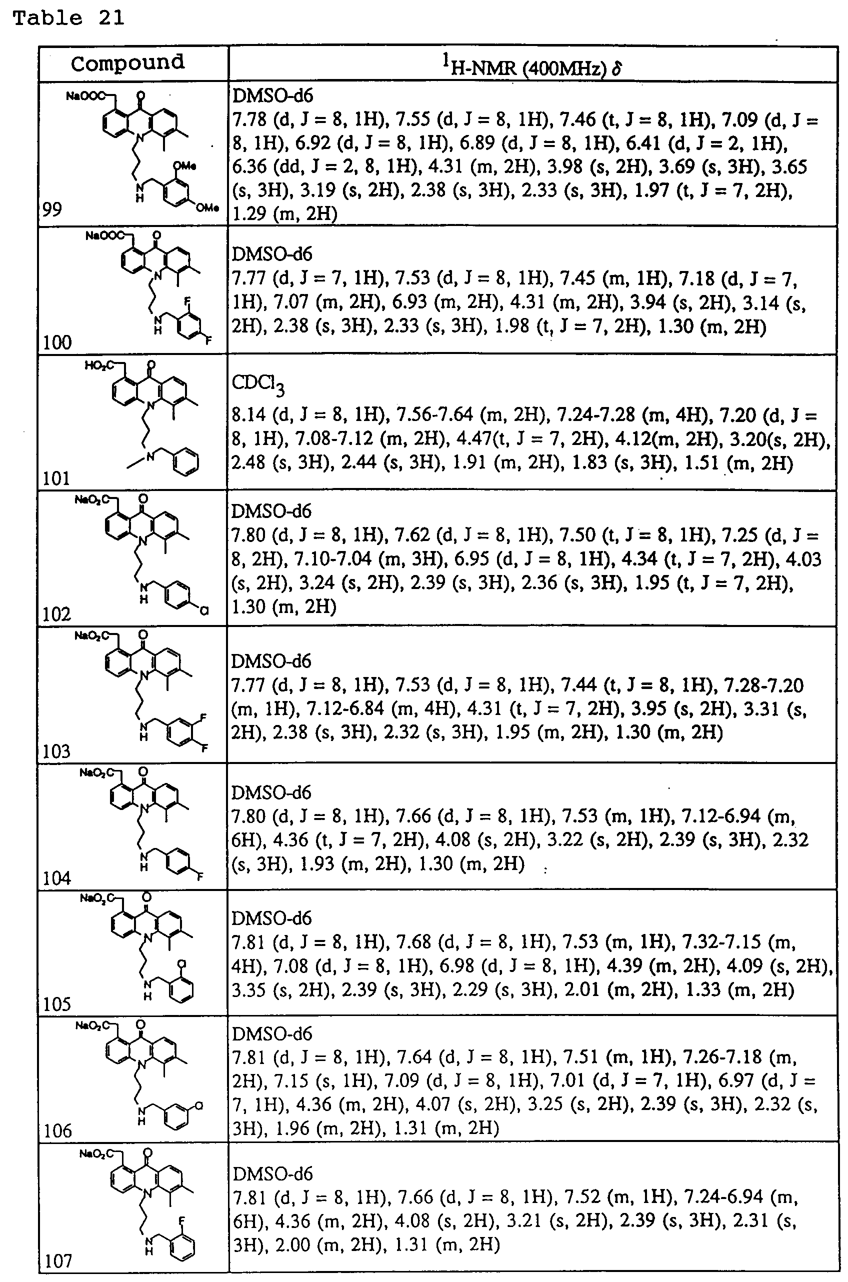

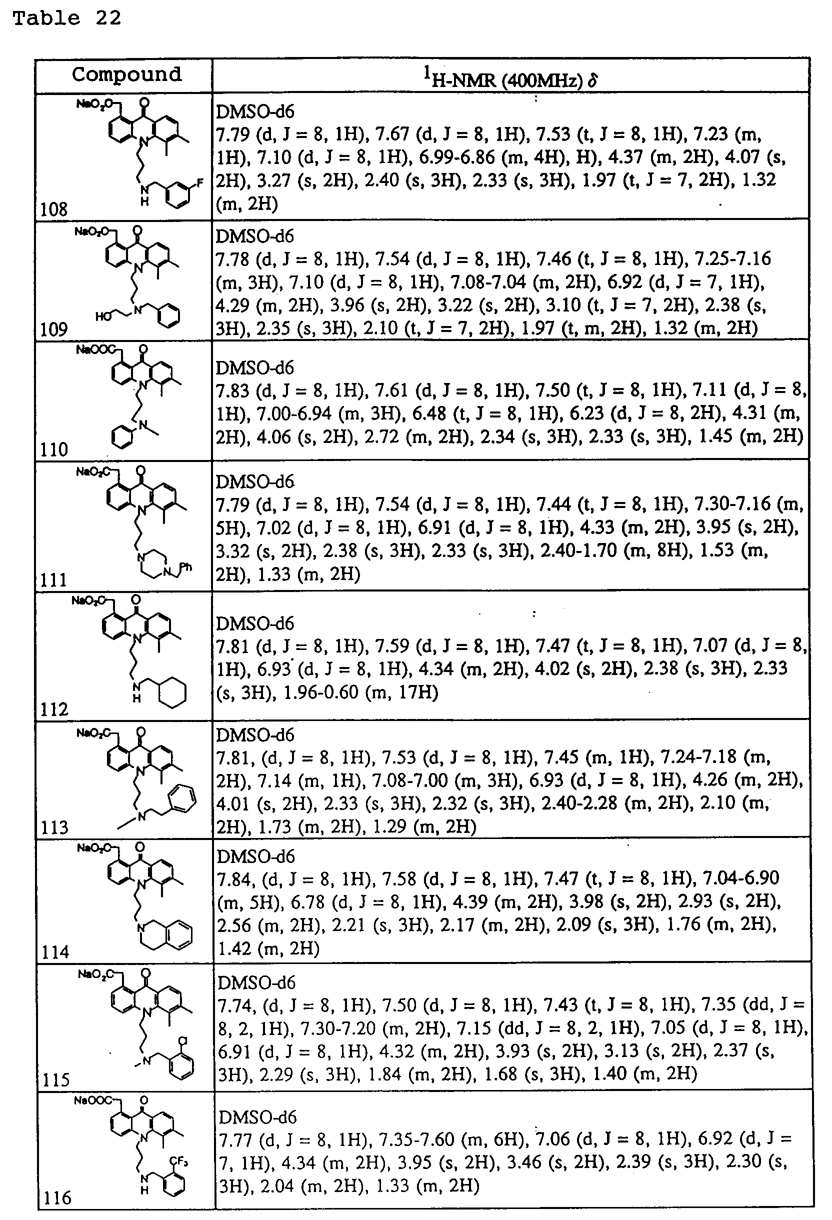

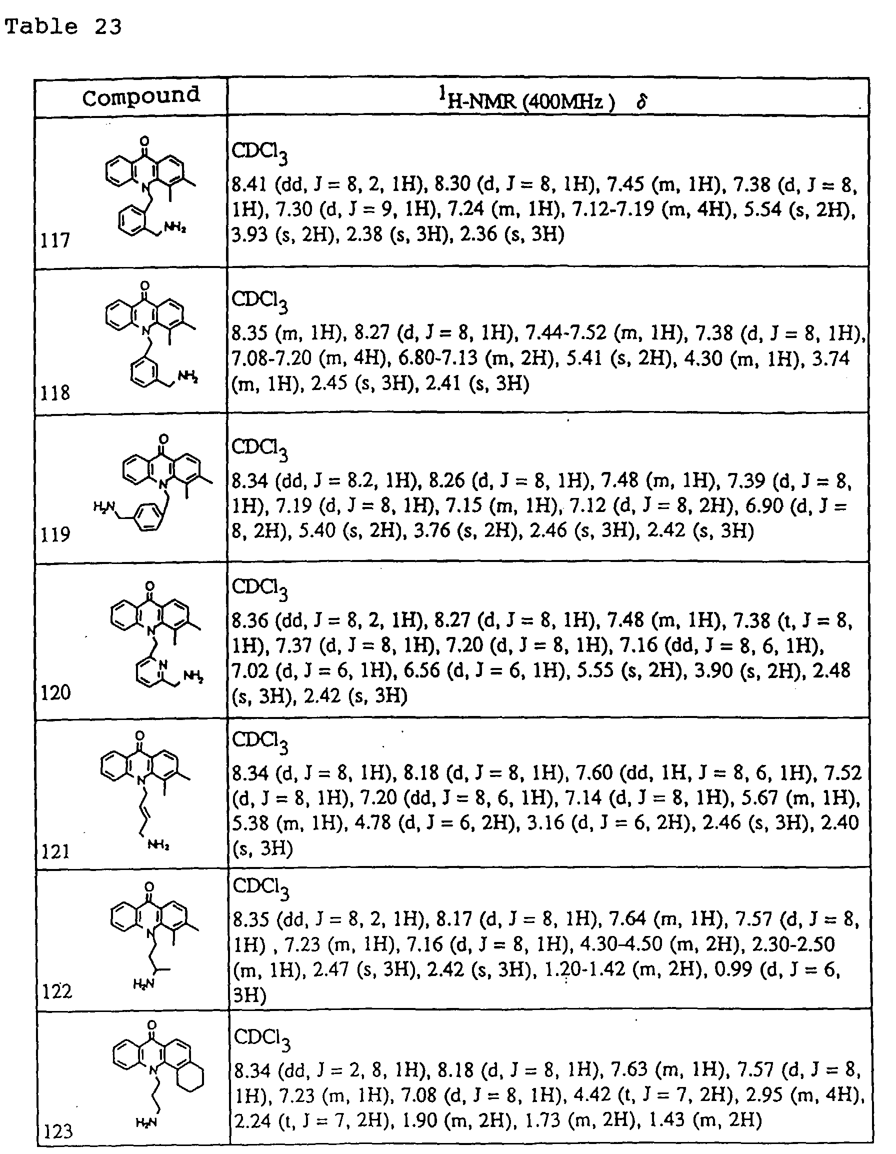

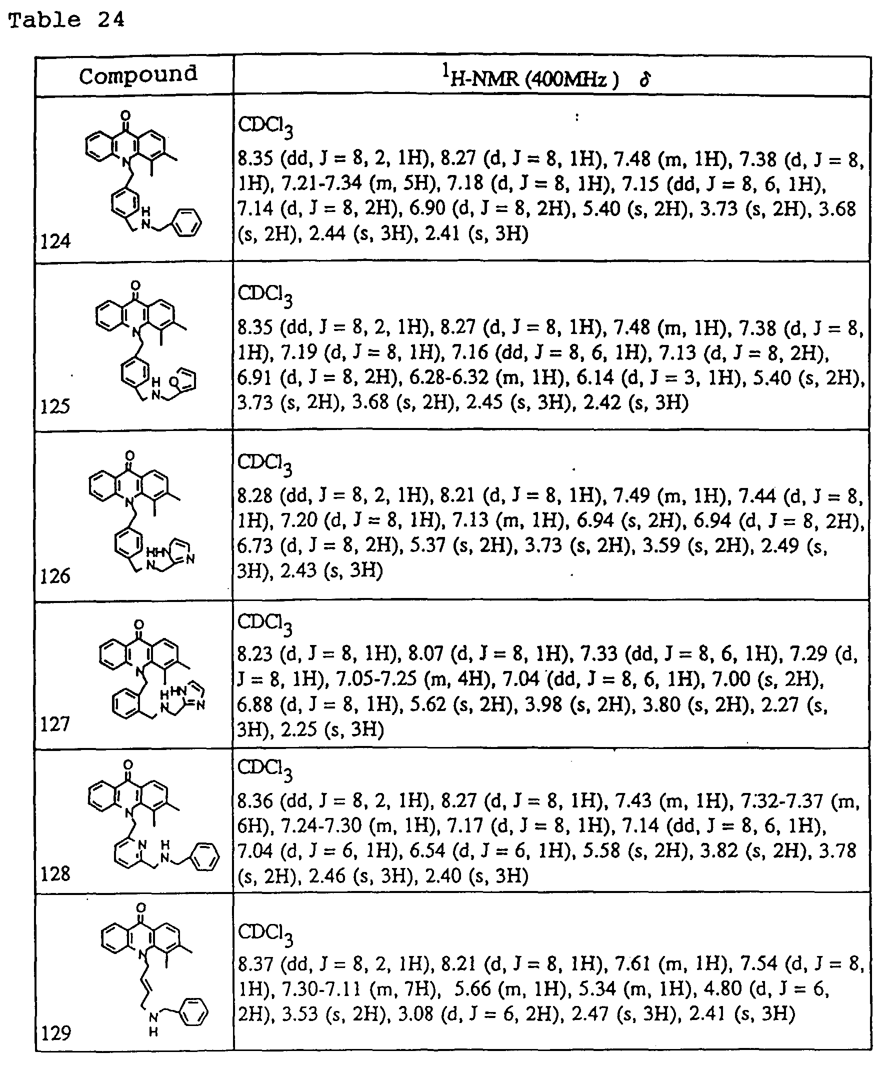

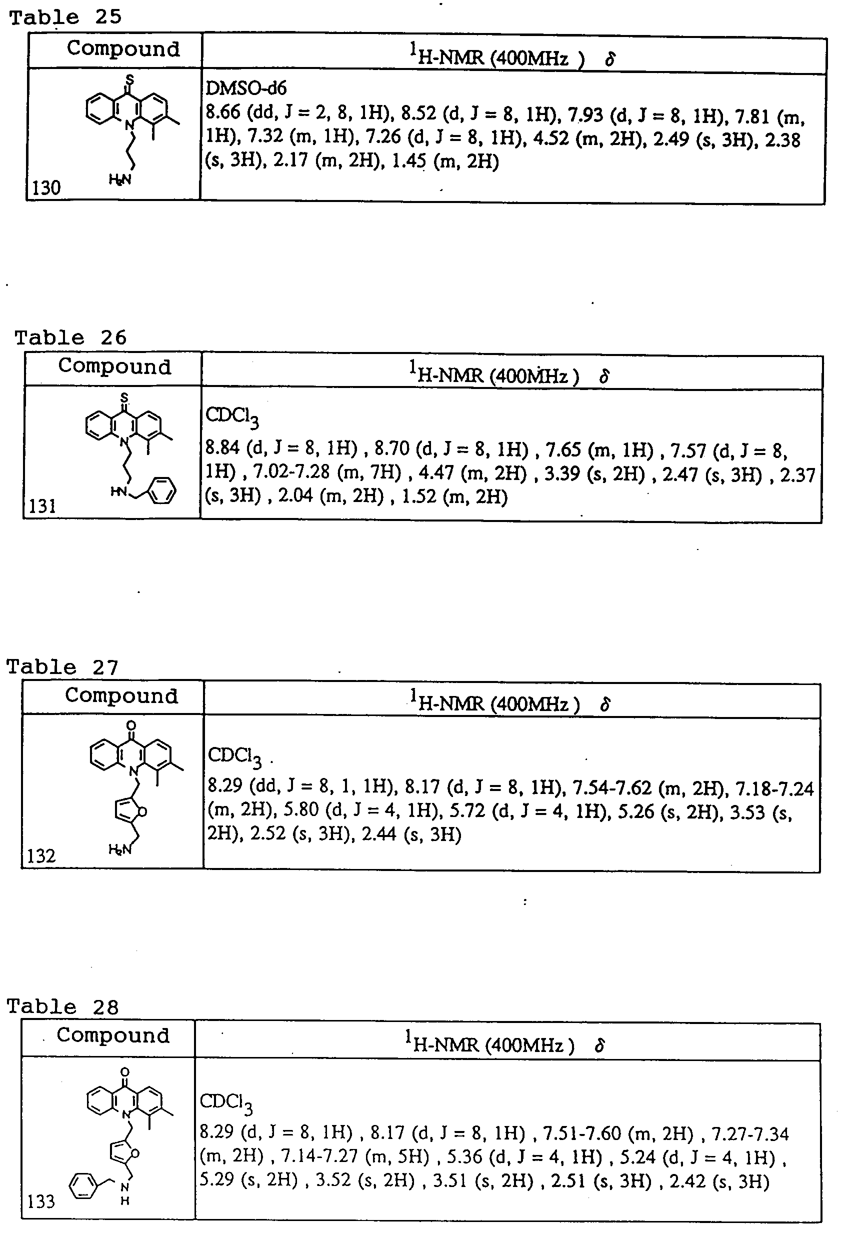

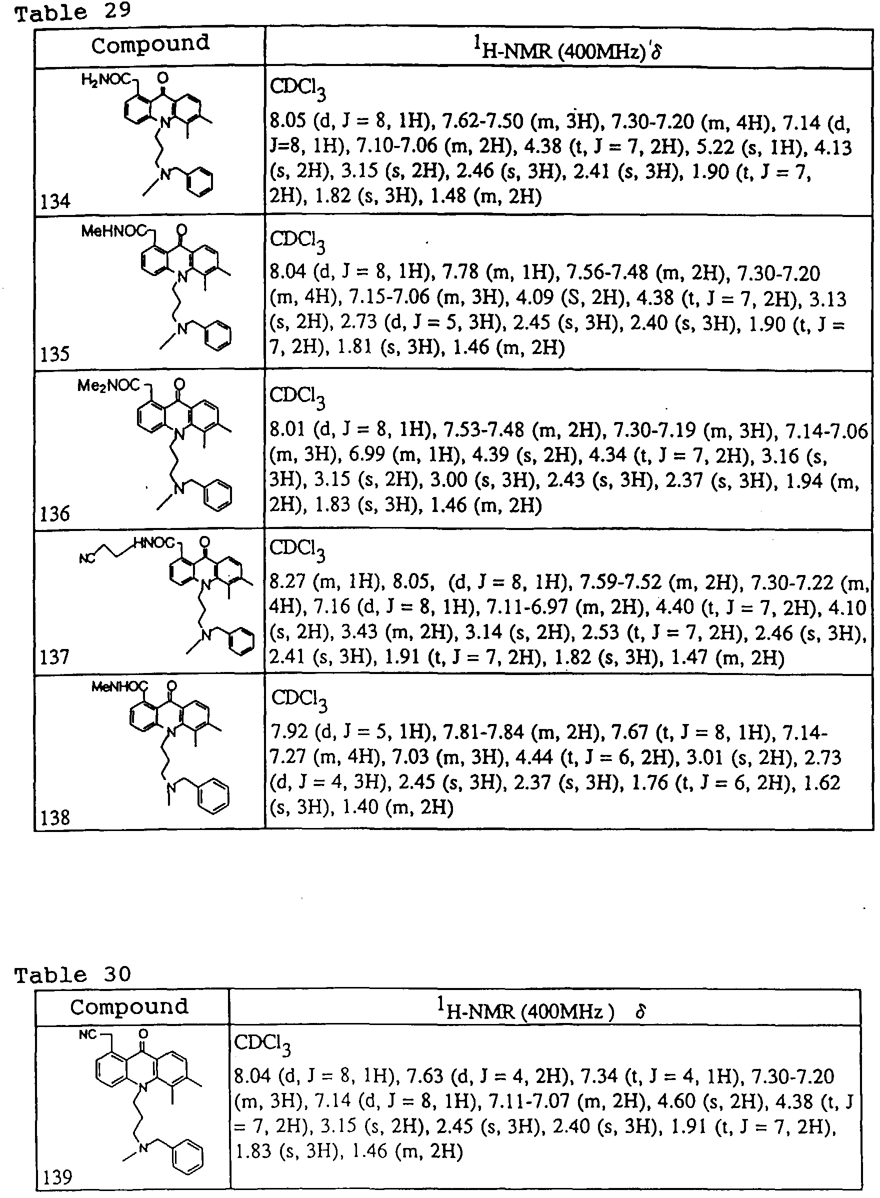

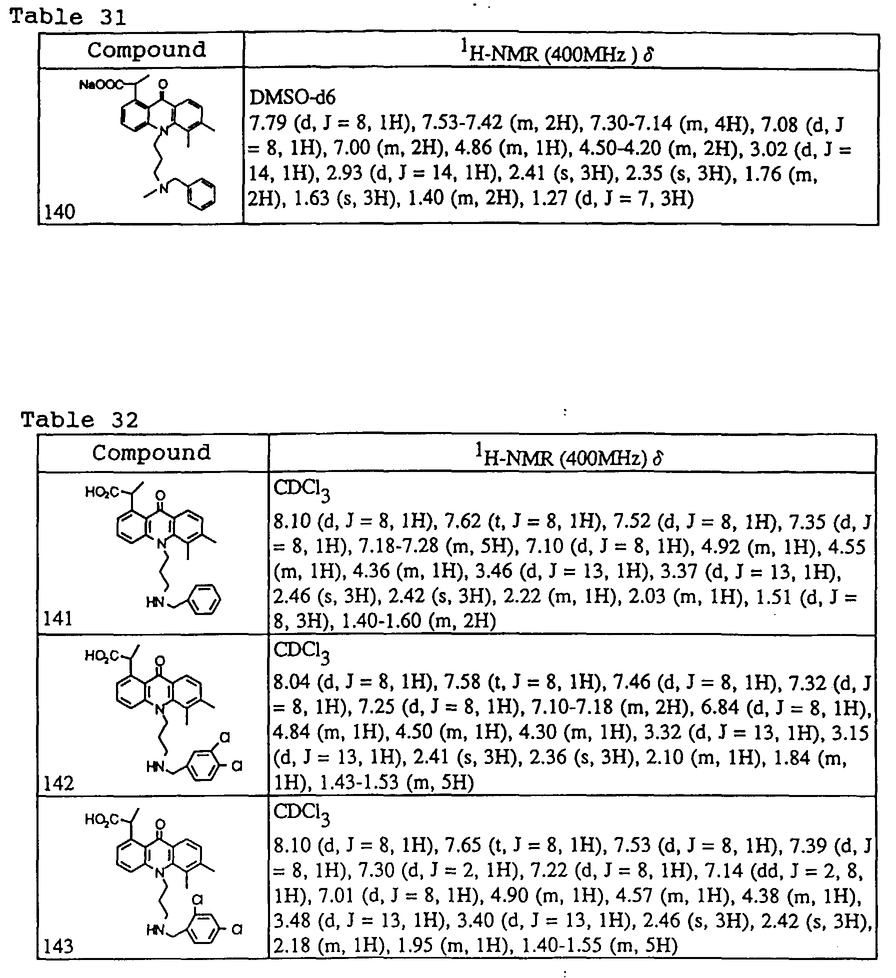

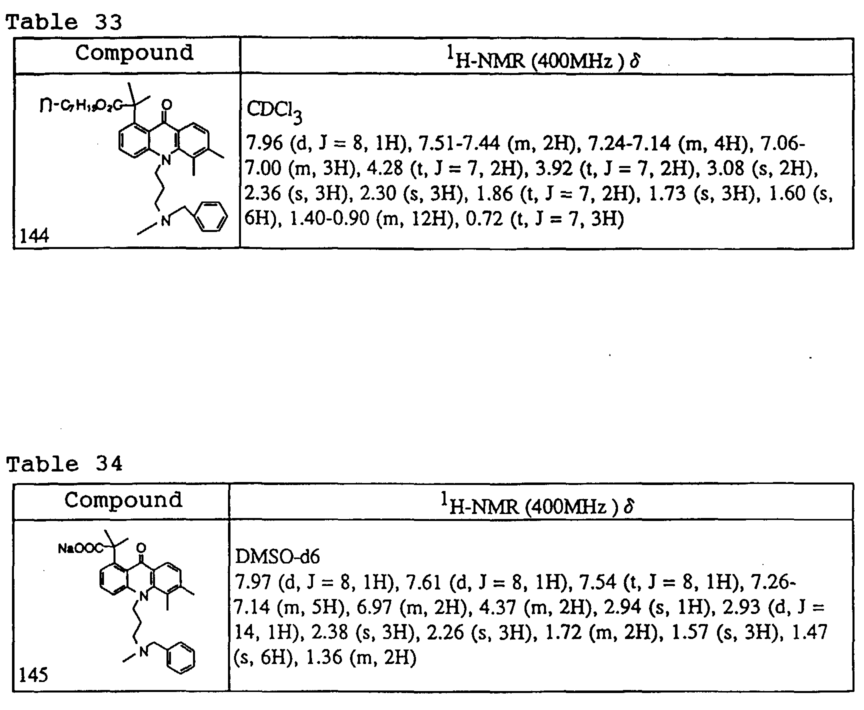

## Claims

1. Acridone compounds represented by the following general formula (I) or pharmaceutically acceptable salts thereof: 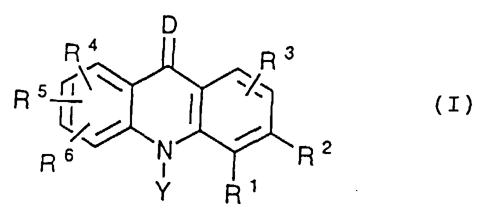 wherein R¹, R², R³, R⁴, R⁵ and R⁶ are the same or different and each represents hydrogen, hydroxy, cyano, nitro, amino, halogeno, lower alkyl, cycloalkyl, cycloalkylalkyl, lower alkoxy, cycloalkyloxy, optionally protected carboxy, optionally substituted heteroaryl, acyl, acylamino, optionally substituted carbamoyl, optionally substituted sulfamoyl, -S(O)ₚ-R⁹ (wherein R⁹ represents hydrogen, lower alkyl or cycloalkyl; and p is an integer of 0 to 2), hydroxyalkyl, alkoxyalkyl, optionally protected and optionally substituted carboxyalkyl, optionally substituted carbamoylalkyl, optionally substituted sulfamoylalkyl, optionally substituted heteroarylalkyl, cyanoalkyl, acylalkyl, acylaminoalkyl, lower alkenyl, hydroxyalkenyl, alkoxyalkenyl, optionally protected carboxyalkenyl, optionally substituted heteroarylalkenyl, cyanoalkenyl, acylalkenyl, acylaminoalkenyl, lower alkynyl, hydroxyalkynyl, alkoxyalkynyl, optionally protected carboxyalkynyl, optionally substituted heteroarylalkynyl, cyanoalkynyl, acylalkynyl, -W-S(O)_{q}-R¹⁰ (wherein R¹⁰ represents hydrogen, lower alkyl or cycloalkyl; W represents alkylene, alkenylene or alkynylene; and q is an integer of 0 to 2), acylaminoalkynyl, hydroxyalkoxy, alkoxyalkoxy, optionally protected carboxyalkoxy, optionally substituted heteroarylalkoxy, cyanoalkoxy, acylalkoxy, acylaminoalkoxy, or -V-S(O)ᵣ-R¹¹ (wherein R¹¹ represents hydrogen, lower alkyl or cycloalkyl; r is an integer of 0 to 2; and V represents alkyleneoxy),
or two of the substituents R¹, R², R³, R⁴, R⁵ and R⁶ adjacent to each other may form together with the carbon atom to which they are bonded a ring which optionally contains as ring-member atom(s) at least one atom selected from the group consisting of nitrogen, sulfur and oxygen and optionally has substituent(s);
Y represents -(CH₂)ₜ-(B)ₘ-(CH₂)ₙ-Z {wherein m is 0 or 1; t and n represent each an integer of 0 to 6; B represents lower alkylene, lower alkenylene, optionally substituted arylene or optionally substituted heteroarylene; and Z represents cyano, optionally protected carboxy, optionally substituted carbamoyl, acyl, acylalkyl or -NR⁷R⁸ [wherein R⁷ and R⁸ are the same or different and each represents hydrogen, lower alkyl, lower alkoxy, hydroxyalkyl, alkoxyalkyl, optionally substituted aryl, optionally substituted arylalkyl, optionally substituted heteroaryl, optionally substituted heteroarylalkyl, optionally substituted aryloxy, optionally substituted arylalkoxy, optionally substituted heteroaryloxy, optionally substituted heteroarylalkoxy, optionally protected and optionally substituted carboxyalkyl, acyl, optionally substituted acylamino, optionally substituted acylaminoalkyl, -S(O)ₛ-(X)ᵤ-R¹² (wherein R¹² represents hydrogen, lower alkyl or cycloalkyl; X represents alkylene; s is an integer of 0 to 2; and u is 0 or 1), aminoalkyl, cyanoalkyl, acylalkyl, cycloalkyl, cycloalkylalkyl or amidino optionally substituted by lower alkyl at the N-position, or R⁷ and R⁸ may form together with the nitrogen atom to which they are bonded a ring which optionally contains at least one ring-member factor selected from the group consisting of nitrogen, sulfur, oxygen and >NR⁹ (wherein R⁹ represents hydrogen, lower alkyl, aryl or arylalkyl) and optionally has substituent(s)]}; and
D represents oxygen or sulfur;
exclusive of the cases where R¹, R², R³, R⁴, R⁵ and R⁶ are the same or different and each represents hydrogen, lower alkyl, lower alkoxy or halogens; Y represents -(CH₂)ₜ-Z {wherein t is an integer of 1 to 6; and Z represents -NR¹R⁸ [wherein R⁷ and R⁸ are the same or different and each represents hydrogen, lower alkyl, unsubstituted aryl, unsubstituted arylalkyl, unsubstituted heteroaryl or unsubstituted heteroarylalkyl, or R⁷ and R⁸ form together with the nitrogen atom to which they are bonded a five- or six-membered ring further containing as ring-member atom(s) oxygen, sulfur or >NR⁹ (wherein R⁹ is as defined above)]}; and D represent oxygen.

2. The acridone compounds as set forth in Claim 1 or pharmaceutically acceptable salts thereof, excluding compounds represented by the general formula (I) and pharmaceutically acceptable salts thereof, wherein R¹, R², R³, R⁴, R⁵ and R⁶ are the same or different and each represents hydrogen, lower alkyl, lower alkoxy or halogeno; Y represents -(CH₂)ₜ-Z {wherein t is an integer of 1 to 6; and Z represents -NR⁷R⁸ [wherein R⁷ and R⁸ are the same or different and each represents hydrogen, lower alkyl, optionally substituted aryl, optionally substituted arylalkyl, optionally substituted heteroaryl or optionally substituted heteroarylalkyl, or R⁷ and R⁸ form together with the nitrogen atom to which they are bonded a five- or six-membered ring further containing as ring-member atom(s) oxygen, sulfur or- >NR⁹ (wherein R⁹ is as defined above)]}; and D represent oxygen.

3. The acridone compounds as set forth in Claim 1 or pharmaceutically acceptable salts thereof, wherein R¹ and R² are the same or different and each represents lower alkyl or R¹ and R² form together with the carbon atom to which they are bonded a ring which optionally contains as ring-member atom(s) at least one atom selected from the group consisting of nitrogen, sulfur and oxygen and optionally has substituent(s).

4. The acridone compounds as set forth in Claim 1 or pharmaceutically acceptable salts thereof, wherein R¹ and R² are both methyl or one of them is methyl while another is lower alkyl.

5. The acridone compounds as set forth in Claim 1 or pharmaceutically acceptable salts thereof, wherein D is oxygen.

6. The acridone compounds as set forth in Claim 1 or pharmaceutically acceptable salts thereof, wherein D is sulfur.

7. A acridone compounds as set forth in Claim 1 or pharmaceutically acceptable salts thereof, wherein R¹ and R² are both methyl and Y is -(CH₂)ₜ-Z {wherein t is an integer of 1 to 3; and Z represents -NR⁷R⁸ [wherein R⁷ and R⁸ are each as defined above]}.

8. The acridone compounds as set forth in Claim 1 or pharmaceutically acceptable salts thereof, wherein at least one of R⁴, R⁵ and R⁶ is optionally protected carboxy, optionally protected and optionally substituted carboxyalkyl, optionally protected carboxyalkenyl or optionally protected carboxyalkynyl.

9. The acridone compounds as set forth in Claim 1 or pharmaceutically acceptable salts thereof, wherein at least on of R⁴, R⁵ and R⁶ is optionally protected carboxy or optionally protected and optionally substituted carboxyalkyl.

10. The acridone compounds as sat forth in Claim 1 or pharmaceutically acceptable salts thereof, wherein y represents -(CH₂)ₜ-(B)ₘ-(CH₂)ₙ-Z {wherein B, m, n and t are each as defined above; and Z represents -NR⁷R⁸ [wherein one of R⁷ and R⁸ is hydroxylated arylalkyl and another is as defined above]}.

11. The acridone compounds as set forth in Claim 1 or pharmaceutically acceptable salts thereof, wherein R¹ and R² are both methyl; and at least one of R⁴, R⁵ and R⁶ is optionally protected carboxy or optionally protected and optionally substituted carboxyalkyl.

12. The acridone compounds as set forth in Claim 1 or pharmaceutically acceptable salts thereof, wherein R¹ and R² are both methyl; and R⁴ is optionally protected carboxy or optionally protected and optionally substituted carboxyalkyl.

13. The aridone compounds as set forth in Claim 1 or pharmaceutically acceptable salts thereof, wherein R¹ and R² are both methyl; R⁴ is optionally protected carboxy or optionally protected and optionally substituted carboxyalkyl; and Y represents -(CH₂)ₜ-Z {wherein t is 2 or 3; and represents -NR⁷R⁸ [wherein one of R⁷ represents hydrogen or methyl; and R⁸ represents optionally substituted arylalkyl or optionally substituted heteroarylalkyl]}.

14. The acridone compounds as set forth in Claim 1 or pharmaceutically acceptable salts thereof, wherein R¹ and R² are both methyl; R⁴ is optionally protected and optionally substituted carboxyalkyl; and Y represents -(CH₂)₃-Z {wherein Z represents -NR⁷R⁸ [wherein one of R⁷ represents hydrogen or methyl; and R⁸ represents optionally substituted arylalkyl or optionally substituted heteroarylalkyl]}.

15. The acridone compounds as set forth in Claim 1 or pharmaceutically acceptable salts thereof, wherein R¹ and R² are both methyl; R³, R⁵ and R⁶ are each hydrogen; R⁴ is optionally protected and optionally substituted carboxymethyl; and Y represents -(CH₂)₃-Z {wherein Z represents -NR⁷R⁸ [wherein one of R⁷ represents hydrogen or methyl; and R⁸ represents optionally substituted arylalkyl or optionally substituted heteroarylalkyl]}.

16. Agents for preventing an IgE receptor γ-chain from binding to a tyrosine kinase of 72 kDa, which contain as the active ingredient acridone compounds as set forth in Claim 1 or pharmaceutically acceptable salts thereof.

17. Preventives and remedies for diseases against which the effect of preventing an IgE receptor γ-chain from binding to a tyrosine kinase of 72 kDa is efficacious, which contain as the active ingredient acridone compounds as set forth in Claim 1 or pharmaceutically acceptable salts thereof.

18. Preventives and remedies for diseases against which an antiallergic effect is efficacious, which contain as the active ingredient acridone compounds as set forth in Claim 1 or pharmaceutically acceptable salts thereof.

19. Preventives and remedies for allergic diseases containing as the active ingredient acridone compounds as set forth in Claim 1 or pharmaceutically acceptable salts thereof.

20. Preventives and remedies for allergic rhinitis, atopic dermatitis, urticaria, hay fever, digestive tract allergy, food allergy or asthma, which contain as the active ingredient acridone compounds as set forth in Claim 1 or pharmaceutically acceptable salts thereof.

21. Medicinal compositions comprising acridone compounds as set forth in Claim 1 or pharmaceutically acceptable salts thereof in an efficacious amount for preventing or treating diseases against which an antiallergic effect is efficacious and pharmacological carriers.

22. Methods for preventing or treating diseases against which an antiallergic effect is efficacious, which comprises administering to a patient acridone compounds as set forth in Claim 1 or pharmaceutically acceptable salts thereof in an efficacious dose for preventing or treating the same.
